(19) Europäisches Patentamt
European Patent Office
Office européen des brevets

(11) Veröffentlichungsnummer: **0 336 199**
**A1**

(12) # EUROPÄISCHE PATENTANMELDUNG

(21) Anmeldenummer: 89104995.9

(22) Anmeldetag: 21.03.89

(51) Int. Cl.⁴: C07D 213/32 , C07D 213/64 , C07D 213/70 , C07D 213/65 , C07D 239/34 , C07D 239/38 , C07D 237/14 , C07D 237/18 , C07D 253/06 , A01N 55/00 , A01N 43/00

Claims for the following Contracting States: ES.

(30) Priorität: 26.03.88 DE 3810379

(43) Veröffentlichungstag der Anmeldung: 11.10.89 Patentblatt 89/41

(84) Benannte Vertragsstaaten: AT BE CH DE ES FR GB GR IT LI NL

(71) Anmelder: **HOECHST AKTIENGESELLSCHAFT**
**Postfach 80 03 20**
**D-6230 Frankfurt am Main 80(DE)**

(72) Erfinder: **Schubert, Hans Herbert, Dr.**
**Geisenheimer Strasse 95**
**D-6000 Frankfurt am Main 71(DE)**
Erfinder: **Salbeck, Gerhard, Dr.**
**Frankfurter Strasse 34**
**D-6238 Hofheim am Taunus(DE)**
Erfinder: **Krause, Hans-Peter, Dr.**
**Berliner Strasse 10**
**D-6238 Hofheim am Taunus(DE)**
Erfinder: **Knauf, Werner, Dr.**
**Im Kirschgarten 24**
**D-6239 Eppstein/Taunus(DE)**
Erfinder: **Waltersdorfer, Anna, Dr.**
**Rauenthaler Weg 28**
**D-6000 Frankfurt am Main 71(DE)**
Erfinder: **Kern, Manfred, Dr.**
**Im Traminer Weg 8**
**D-6501 Lörzweiler(DE)**

(54) **Azaneophyl- und Silazaneophylsulfide, Verfahren zu ihrer Herstellung, sie enthaltende Mittel und ihre Verwendung als Schädlingsbekämpfungsmittel.**

(57) Verbindungen der Formel I, ihre optischen Isomeren und deren Gemische

$$R^1-\underset{R^3}{\overset{R^2}{M}}-CH_2-S-\underset{R^4}{CH}-R^5 \qquad (I),$$

worin
M = C oder Si,
$R^1$ = unsubstituiertes oder substituiertes Pyridyl, unsubstituiertes oder substituiertes Pyrimidyl, sowie - für M = C- unsubstituiertes oder substituiertes Pyridazinyl, unsubstituiertes oder substituiertes Pyrazinyl, unsub-

EP 0 336 199 A1

stituiertes oder substituiertes 1,2,4-Triazinyl, unsubstituiertes oder substituiertes 1,2,4,5-Tetrazinyl,
$R^2$, $R^3$ = unabhängig voneinander Alkyl, Alkenyl, Haloalkyl, Phenyl oder $R^2$ und $R^3$ eine Alkylenkette, die - zusammen mit dem quartären Zentralatom (M)-einen unsubstituierten oder fluorsubstituierten Ring mit vier bis sechs Ringgliedern (für M = Si) oder mit drei bis sechs Ringgliedern (für M = C) ergibt,
$R^4$ = H, F, -CN, $-CCl_3$, -C≡CH, $(C_1-C_4)$Alkyl, $-\underset{S}{\underset{\|}{C}}-NH_2$,
$R^5$ = Pyridyl, Furyl, Thienyl, die alle substituiert sein können, Phthalimidyl, Dialkylmaleinimidyl, Thiophthalimidyl, Dihydrophthalimidyl, Tetrahydrophthalimidyl, substituiertes Phenyl
oder $R^4$ und $R^5$ - zusammen mit dem sie verbrückenden Kohlenstoffatom - einem gegebenenfalls substituierten Indanyl-, Cyclopentenoyl- oder Cyclopentenyl-Rest
bedeuten, sowie deren Salze und Quaternisierungsprodukte besitzen vorteilhafte insektizide, akarizide oder nematozide Eigenschaften.

# Azaneophyl- und Silazaneophylsulfide, Verfahren zu ihrer Herstellung, sie enthaltende Mittel und ihre Verwendung als Schädlingsbekämpfungsmittel

Die insektizide und akarizide Wirkung bestimmter Silazaneophylether und ihrer Kohlenwasserstoffanaloga ist in der EP-A 0249015 beschrieben. Ferner sind aus der EP-A 0 224 024 schwefelhaltige Silanderivate bekannt, die ebenfalls insektizide und akarizide Wirksamkeiten aufweisen.

Es wurden nun neue Azaneophyl- und Silazaneophylsulfide gefunden, die vorteilhafte insektizide, akarizide oder nematozide Eigenschaften aufweisen.

Gegenstand der vorliegenden Erfindung sind daher die Verbindungen der Formel (I), ihre optischen Isomeren und deren mögliche Gemische

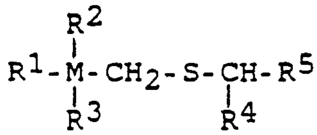

(I),

worin
M = C oder Si,
$R^1$ = unsubstituiertes oder substituiertes Pyridyl, unsubstituiertes oder substituiertes Pyrimidyl, sowie - für M = C- unsubstituiertes oder substituiertes Pyridazinyl, unsubstituiertes oder substituiertes Pyrazinyl, unsubstituiertes oder substituiertes 1,2,4-Triazinyl, unsubstituiertes oder substituiertes 1,2,4,5-Tetrazinyl,
$R^2$, $R^3$ = unabhängig voneinander $(C_1$-$C_3)$Alkyl, $(C_2$-$C_8)$Alkenyl, $(C_1$-$C_2)$Haloalkyl, Phenyl oder $R^2$ und $R^3$ eine Alkylenkette, die - zusammen mit dem quartären Zentralatom (M) - einen unsubstituierten oder fluorsubstituierten Ring mit vier bis sechs Ringgliedern (für M = Si) oder mit drei bis sechs Ringgliedern (für M = C) ergibt,
$R^4$ = H, F, -CN, $-CCl_3$, $-C{\equiv}CH$, $(C_1$-$C_4)$Alkyl, $-\underset{\underset{S}{\|}}{C}-NH_2$,
$R^5$ = Pyridyl, Furyl, Thienyl, die alle substituiert sein können, Phthalimidyl, Di$(C_1$-$C_4)$-alkylmaleinimidyl, Thiophthalimidyl, Dihydrophthalimidyl, Tetrahydrophthalimidyl, substituiertes Phenyl
oder $R^4$ und $R^5$ - zusammen mit dem sie verbrückenden Kohlenstoffatom - einen gegebenenfalls substituierten Indanyl-, Cyclopentenoyl- oder Cyclopentenyl-Rest
bedeuten, sowie deren für landwirtschaftliche Zwecke einsetzbaren Salze und Quaternisierungsprodukte. Die Salzbildung bzw. Quaternisierung erfolgt hierbei nach üblichen Methoden durch Addition geeigneter Verbindungen an das basische Stickstoffatom bzw. die basischen Stickstoffatome der heterocyclischen Reste $R^1$ und/oder $R^5$. Es ist somit eine mehrfache Salzbildung oder Quaternisierung möglich.

Zur Salzbildung am Stickstoff geeignet sind alle anorganischen oder organischen Säuren, die aufgrund ihres pKs-Wertes zur Salzbildung befähigt sind, z.B. Halogenwasserstoffsäuren, Salpetersäure, Schwefelsäure, Phosphorsäure, Phosphonsäure, Sulfonsäure, Halogenessigsäuren oder Oxalsäure.

Als gegebenenfalls substituiertes Pyridyl $R^1$, Pyrimidyl $R^1$, Pyridazinyl $R^1$, Pyrazinyl $R^1$, 1, 2,4-Triazinyl $R^1$ oder 1,2,4,5-Tetrazinyl $R^1$ steht bevorzugt ein Rest der allgemeinen Formeln (A), (B), (C), (D), (E) oder (F),

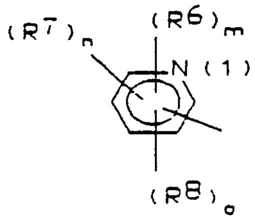

(A)

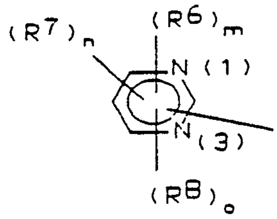

(B)

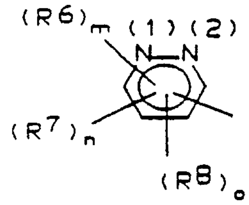

(C)

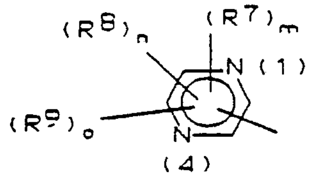

(D)

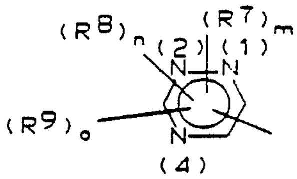

(E)

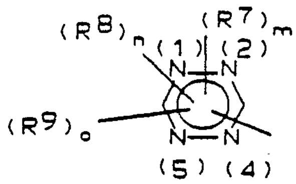

(F)

worin $0 \leq m+n+o \leq 3$ und m, n, o die Werte 0 bis 2 besitzen können. $R^6$, $R^7$, $R^8$ und $R^9$ stehen unabhängig voneinander für $(C_1-C_5)$Alkyl, Halogen, $(C_2-C_6)$Alkenyl, $(C_2-C_6)$Alkinyl, $(C_3-C_7)$Cycloalkyl, $(C_1-C_3)$Alkoxy, $(C_2-C_4)$Alkenyloxy, $(C_2-C_4)$Alkinyloxy, $(C_1-C_4)$Alkylthio, $(C_3-C_6)$Cycloalkyloxy, $(C_1-C_4)$-Halogenalkyl, $(C_1-C_3)$Halogenalkoxy, $(C_1-C_3)$ Halogenalkylthio, $(C_2-C_5)$Halogenalkenyl, $(C_2-C_5)$ Halogenalkenyloxy, $(C_2-C_5)$Halogenalkenylthio oder zwei der Reste $R^6$, $R^7$, $R^8$, $R^9$ bilden - wenn sie orthoständig angeordnet sind - einen Methylendioxy-, Ethylendioxy oder $(C_3-C_5)$Alkylenrest.

Die Verknüpfungsstelle (freie Valenz) des Pyridylrestes (z.B. in Formel (A)) an das quartäre Zentralatom M in Formel (I) ist bevorzugt in 2- oder 3-Stellung des Pyridylrestes (N = Position 1), besonders bevorzugt in 3-Stellung des Pyridylrestes. Der Pyrimidylrest (z.B. Formel (B)) ist bevorzugt in Position 2 oder 5 an das quartäre Zentralatom M gebunden. Die bevorzugte Verknüpfungsstelle des Pyridazinylrestes (z.B. Formel (C)) ist die Position 3, die des Pyrazinylrestes (z.B. Formel (D)) die Position 2. Triazinylreste (z.B. Formel (E)) und Tetrazinylreste (z.B. Formel (F)) sind jeweils bevorzugt in 3- oder 6-Stellung mit dem quartären Zentralatom verknüpft.

Besonders bevorzugt bedeutet $R^1$ ein- oder zweifach substituierte Pyridyl-, Pyrimidyl, Pyridazinyl oder Pyrazinyl-Reste der Formeln (A), (B), (C) oder (D) mit $m+n+o = 1$ oder 2, wobei die Substituenten ($R^6$-$R^9$) insbesondere para- oder meta-ständig zur Verknüpfungsstelle (quartäres Zentralatom M) orientiert sind. $R^1$ = A ist von besonderer Bedeutung.

$R^2$ und $R^3$ stehen bevorzugt für einen $(C_1-C_3)$Alkylrest wie Methyl, Ethyl, i-Propyl und n-Propyl, einen fluorierten Methylrest wie Fluormethyl, Difluormethyl und Trifluormethyl, oder bedeuten bevorzugt - für M = C- zusammen mit dem sie verknüpfenden Kohlenstoffatom einen unsubstituierten oder mono- oder difluorierten Cyclopropylring.

$R^4$ steht bevorzugt für Wasserstoff, Fluor, Cyano oder $(C_1-C_4)$-Alkyl, besonders bevorzugt für Wasserstoff.

Als substituiertes Phenyl $R^5$ steht bevorzugt ein Phenylrest der allgemeinen Formel (G),

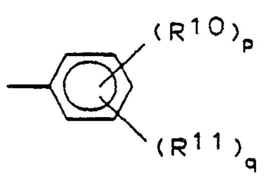

(G)

worin $R^{10}$ und $R^{11}$ - unabhängig voneinander - Halogen, $(C_1-C_4)$Alkyl, $(C_1-C_4)$Alkoxy, $(C_1-C_4)$Halogenalkyl, Phenyl, N-Pyrrolyl oder eine Gruppe der allgemeinen Formel (H) bedeuten kann,

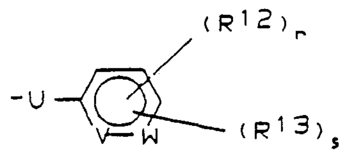

(H)

worin $R^{12}$ und $R^{13}$ = unabhängig voneinander H, Halogen, $(C_1\text{-}C_4)$Alkyl, $(C_1\text{-}C_4)$Alkoxy und $(C_1\text{-}C_4)$-Halogenalkyl; U = $-CH_2-$, C=O, -O- oder -S-, bevorzugt -O-; V, W = CH oder N, wobei beide gleichzeitig CH aber nicht gleichzeitig N bedeuten können,
und wobei in Formeln (G) und (H)
p, q = eine ganze Zahl von 0 bis 5 mit der Bedingung, daß die Summe p + q eine Zahl von 1 bis 5 bedeuten muß,
r, s = 0, 1 oder 2, mit der Bedingung, daß die Summe von r + s = 0, 1 oder 2 sein muß, und der Bedingung,
daß, falls $R^{10}$ oder $R^{11}$ der Gruppierung (H) entspricht, p, q = 0 oder 1 und p + q = 1 oder 2 bedeuten muß.

Von diesen Resten für $R^5$ sind von besonderer Bedeutung Reste der Formel (G), worin $(R^{10})_p$ = H oder 4-Fluor und $(R^{11})_q$ in 3-Stellung des Phenylrest orientiert ist und den Rest

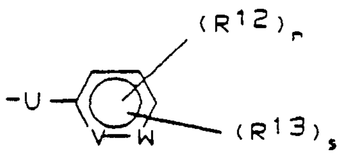

wobei r + s bevorzugt für 0 steht, bedeutet.

Als gegebenenfalls substituiertes Pyridyl $R^5$ steht eine mono- oder disubstituierte Pyridyl-Gruppe der allgemeinen Formel (K)

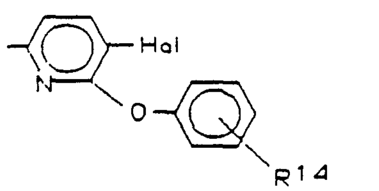

(K)

worin $R^{14}$ = Halogen außer J, $(C_1\text{-}C_4)$Alkyl, $(C_1\text{-}C_4)$Alkoxy oder $(C_1\text{-}C_4)$-Halogenalkyl und Hal = Halogen, insbesondere Fluor, oder H bedeutet.

Als gegebenenfalls substituiertes Thienyl $R^5$ oder Furyl $R^5$ steht ein Heterocyclus der allgemeinen Formel (L)

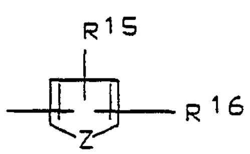

(L)

worin Z = O, S,
$R^{15}$ = H, Halogen, $(C_1\text{-}C_4)$Alkyl, $(C_1\text{-}C_4)$Alkoxy, $(C_1\text{-}C_4)$Halogenalkyl, CN oder $NO_2$ und
$R^{16}$ = gegebenenfalls substituiertes Benzyl, Propargyl, Allyl oder Phenoxy
bedeutet.

Substituierte Phenylreste für $R^5$ sind für die Erfindung von besonderer Wichtigkeit.

Als typische Beispiele für die Gruppe $R^5$ werden folgende Reste angegeben:
Pentafluorphenyl, 5-Benzyl-3-furyl, 4-Phenoxyphenyl, 3-Phenoxyphenyl, 3-(4-Fluorphenoxy)phenyl, 3-(4-Chlorphenoxy) phenyl, 3-(4-Bromphenoxy)phenyl, 3-(3-Fluorphenoxy)phenyl, 3-(3-Chlorphenoxy)phenyl, 3-(3-Bromphenoxy)phenyl, 3-(2-Fluorphenoxy)phenol, 3-(2-Chlorphenoxy)phenyl, 3-(2-Bromphenoxy)phenyl, 3-(4-Methylphenoxy)phenyl, 3-(3-Methylphenoxy)phenol, 3-(2-Methylphenoxy)phenyl, 3-(4-Methoxyphenoxy)phenyl, 3-(3-Methoxyphenoxy)phenyl, 3-(2-Methoxyphenoxy)phenyl, 3-(4-Ethoxyphenoxy)phenyl, 3-(Phenylthio)phenyl, 3-(4-Fluorphenylthio)phenyl, 3-(3-Fluorphenylthio)phenyl, 3-Benzoylphenyl, 3-Benzylphenyl, 3-(4-Fluorbenzyl)phenyl, 3-(4-Chlorbenzyl)phenyl, 3-(3,5-Dichlorphenoxy)phenyl, 3-(3,4-Dichlorphenoxy)phenyl, 3-(4- Chlor-2-methylphenoxy)phenyl, 3-(2-Chlor-5-methylphenoxy)phenyl, 3-(4-Chlor-5-methylphenoxy)phenyl, 3-(4-Ethylenphenoxy)phenyl, 3-(3-Chlor-5-methoxyphenoxy)phenyl, 3-(2,5-Dichlorphenoxy)phenyl, 3-(3,5-Dichlorbenzoyl)phenyl, 3-(3,4-Dichlorbenzoyl)phenyl, 3-(4-Methylbenzyl)phenyl, 3-(4-Isopropoxyphenoxy)phenyl, 4-Fluor-3-phenoxyphenyl, 4-Chlor-3-phenoxyphenyl, 4-Brom-3-phenoxyphenyl, 4-Fluor-3-(4-fluorphenoxy)phenyl, 4-Fluor-3-(4-chlorphenoxy)phenyl, 4-Fluor-3-(4-bromphenoxy)phenyl, 4-Fluor-3-(4-methylphenoxy)phenyl, 4-Fluor-3-(4-methoxyphenoxy)phenyl, 4-Fluor-3-(3-fluorphenoxy)phenyl, 4-Fluor-3-(3-chlorphenoxy)phenyl, 4-Fluor-3-(3-bromphenoxy)phenyl, 4-Fluor-3-(3-methoxyphenoxy)phenyl, 4-Fluor-3-(4-ethoxyphenoxy)phenyl, 4-Fluor-3-(2-fluorphenoxy)phenyl, 3-Methoxy-5-phenoxyphenyl, 2-Fluor-3-phenoxyphenyl, 2-Fluor-3-(4-fluorphenoxy)phenyl, 2-Fluor-3-(3-fluorphenoxy)phenyl, 2-Fluor-3-(2-fluorphenoxy)phenyl, 3-Fluor-5-(4-fluorphenoxy)phenyl, 3-Fluor-5-(3-fluorphenoxy)phenyl, 3-Fluor-5-(2-fluorphenoxy)-phenyl, 4-Methyl-3-phenoxyphenol, 3-Fluor-5-(4-methylphenoxy)phenyl, 3-Fluor-5-(3-methoxyphenoxy)-phenyl, 2-Fluor-5-(4-fluorphenoxy)phenyl, 2-Fluor-5-(3-fluorphenoxy)phenyl, 2-Fluor-5-(2-fluorphenoxy)-phenyl, 2-Chlor-3-phenoxyphenyl, 3-Fluor-5-phenoxyphenyl, 2-Fluor-5-phenoxyphenyl, 2-Chlor-5-phenoxyphenyl, 2-Brom-5-phenoxyphenyl, 4-Chlor-3-(3-methylphenoxy)phenyl, 4-Chlor-3-(4-fluorphenoxy)phenyl, 3-Chlor-5-phenoxyphenyl, 3-Brom-5-phenoxyphenyl, 4-Brom-3-phenoxyphenyl, 4-Trifluormethyl-3-phenoxyphenyl, 4-Fluor-3-phenylthiophenyl, 4-Fluor-3-benzylphenyl, 3-(2-Pyridyloxy)phenyl, 3-(3-Pyridyloxy)-phenyl, 4-Fluor-3-(2-pyridyloxy)phenyl, 4-Chlor-3-(2-pyridyloxy)phenyl, 4-Brom-3-(2-pyridyloxy)phenyl, 4-Methyl-3-(2-pyridyloxy)phenyl, 4-Fluor-3-(3-pyridyloxy)phenyl, 4-Chlor-3-(3-pyridyloxy)phenyl, 4-Brom-3-(3-pyridyloxy)phenyl, 4-Methyl-3-(3-pyridyloxyphenyl), 2-Methyl-3-phenylphenyl, 2-Methyl-3-(N-pyrrolyl)phenyl, 6-Phenoxy-2-pyridyl, 6-(4-Fluorphenoxy)-2-pyridyl, 6-(4-Chlorphenoxy)-2-pyridyl, 6-(4-Bromphenoxy)- 2-pyridyl, 6-(4-Methylphenoxy)-2-pyridyl, 6-(4-Methoxyphenoxy)-2-pyridyl, 6-(4-Ethoxyphenoxy)-2-pyridyl, 6-(3-Fluorphenoxy)-2-pyridyl, 6-(3-Chlorphenoxy)-2-pyridyl, 6-(3-Bromphenoxy)-2-pyridyl, 6-(3-Methoxyphenoxy)-2-pyridyl, 6-(2-Fluorphenoxy)-2-pyridyl, 6-(2-Chlorphenoxy)-2-pyridyl, 6-(2-Bromphenoxy)-2-pyridyl, 5-Propargyl-3-furyl, N-Phthalimidyl, N-3,4,5,6-phthalimidyl, 2-Methyl-5-propargyl-3-furyl, 4-t-Butylphenyl, 4-Methylphenyl, 4-Isopropylphenyl, 4-(2-Chlor-4-trifluormethyl-2-pyridyloxy)phenyl, 4-Cyclohexylphenyl, 4-Difluormethoxyphenyl, 4-Biphenylyl, 4-Trimethylsilylphenyl und 4-Phenoxy-2-thienyl.

Weitere typische Beispiele für die Gruppe

$$-\underset{\displaystyle R^4}{\underset{|}{CH}}-R^5$$

sind:
2-Allyl-3-methylcyclopent-2-en-1-on-4-yl, 4-Phenylindan-2-yl. $R^6$-$R^9$ stehen unabhängig voneinander insbesondere für Methyl, Ethyl, Propyl, iso-Propyl, Butyl, t-Butyl, F, Cl, Br, J, Vinyl, Allyl, Ethinyl, Propargyl, Methoxy, Ethoxy, Propoxy, iso-Propoxy, Difluormethoxy, Bromdifluormethoxy, Chlordifluormethoxy, Trifluormethoxy, 1,1,2,2-Tetrafluorethoxy, 2-Chlor-1,1,2-trifluorethoxy, 2,2,2-Trifluorethoxy, 2-Fluorethoxy, 1,1,2,3,3,3-Hexafluorpropoxy, Vinyloxy, Allyloxy, Propargyloxy, Ethylthio, Methylthio, Propylthio, iso-Butylthio, Trifluormethyl, 1,1,2,2-Tetrafluorethyl, 1,1,2,2-Tetrafluorethylthio, 2,2,2-Trifluorethylthio, Difluormethylthio, Trifluormethylthio, Chlordifluormethylthio, Bromdifluormethylthio, Heptafluorpropyl, Trichlorvinyl, Trichlorvinyloxy, Methylendioxy oder Ethylendioxy.

Gegenstand der vorliegenden Erfindung sind auch Verfahren zur Herstellung der Verbindungen der allgemeinen Formel (I), dadurch gekennzeichnet, daß man

a) ein Mercaptan der allgemeinen Formel (II) oder dessen Salz der allgemeinen Formel III,

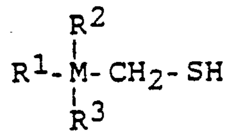

(II)

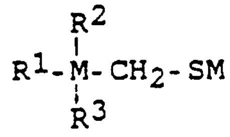

(III)

worin M′ einem Alkalimetall- oder Erdalkalimetall-Äquivalent, insbesondere Li, Na, K, Mg, Ca entspricht, mit einem Alkylierungsmittel der allgemeinen Formel (IV),

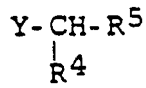

(IV)

worin Y eine nucleofuge Abgangsgruppe wie beispielsweise Halogen oder Sulfonat bedeutet, gegebenenfalls in Gegenwart einer Base,
oder

b) ein Mercaptan der allgemeinen Formel (V) oder dessen Salz der allgemeinen Formel (VI)

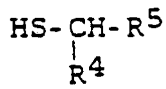

(V)

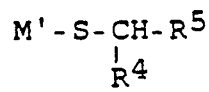

(VI)

mit einem Alkylierungsmittel der allgemeinen Formel (VII),

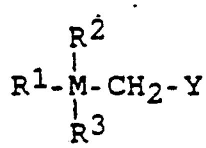

(VII)

gegebenenfalls in Gegenwart einer Base,
oder

c) ein Sulfid der allgemeinen Formel (VIII) oder (IX)

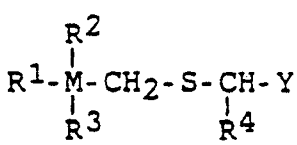

(VIII)

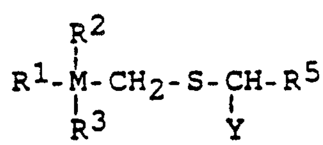

(IX)

mit einer Metallverbindung der allgemeinen Formel (X) oder (XI)

$M'-R^5$

(X)

$M'-R^4$

(XI)

oder - für M = Si -

d) ein Silan der allgemeinen Formel (XII)

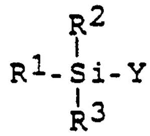

(XII)

mit einer metallorganischen Verbindung der allgemeinen Formel (XIII)

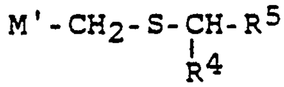

(XIII)

oder - für M = Si -

e) eine metallorganische Verbindung der allgemeinen Formeln (XIV), (XV) oder (XVI)

| | | |
|---|---|---|
| $R^1$-M' | $R^2$M' | $R^3$M' |
| (XIV) | (XV) | (XVI) |

mit einem Silan der allgemeinen Formeln (XVII), (XVIII) oder (XIX)

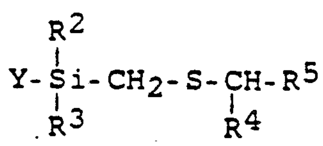

(XVII)

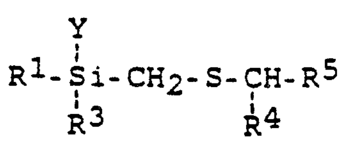

(XVIII)

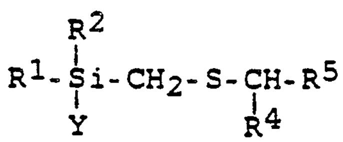

(XIX)

umsetzt.

Die als Ausgangsverbindung beim Herstellungsverfahren a) zu verwendenden Mercaptane der allgemeinen Formel (II) sind zum Teil neu und können nach einem an sich literaturbekannten Verfahren hergestellt werden, indem man z.B. geeignete Alkylierungsmittel der allgemeinen Formel (XX) (siehe EP-A 0249015 und DE-P 37 12 752.7)

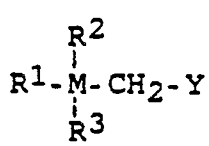

(XX)

zunächst mit einem Schwefelnucleophil wie Disulfid, Thiosulfat, Thioacetat oder Thioharnstoff umsetzt und die entstehenden Zwischenprodukte der allgemeinen Formeln (XXI), (XXII), (XXIII) oder (XXIV) nach den

üblichen Standardmethoden (siehe auch Methoden der org. Chemie (Houben-Weyl), Bd. E11/1, Georg Thieme Verlag, Stuttgart 1985; S. 32 ff) verseift oder reduziert

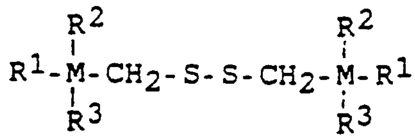
(XXI)

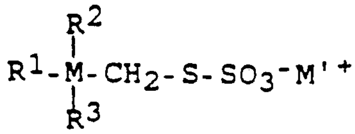
(XXII)

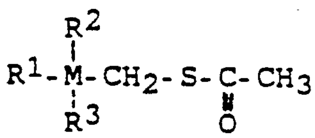
(XXIII)

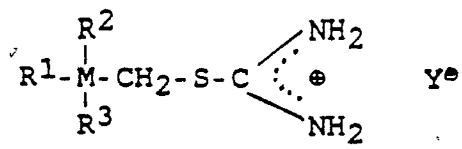
(XXIV).

Die als Ausgangsverbindungen beim Herstellungsverfahren c) zu verwendenden Sulfide (VIII) und (IX) sind z.T. neu und können nach einem an sich literaturbekannten Verfahren hergestellt werden, indem man ein Alkylierungsmittel der allgemeinen Formeln (XXV) oder (XXVI)

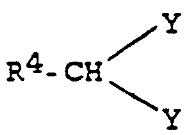
(XXV)

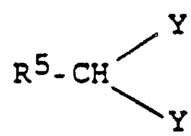
(XXVI)

mit zwei Äquivalenten eines Mercaptans der Formel (II) oder dessen Salz der Formel (III), gegebenenfalls in Gegenwart einer Base, umsetzt und die erhaltenen Zwischenstufen der allgemeinen Formel (XXVII) oder (XXVIII)

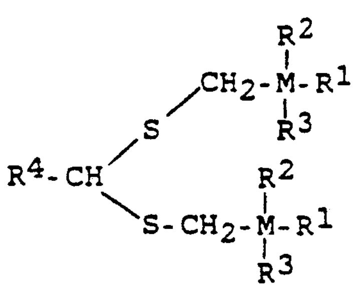
(XXVII)

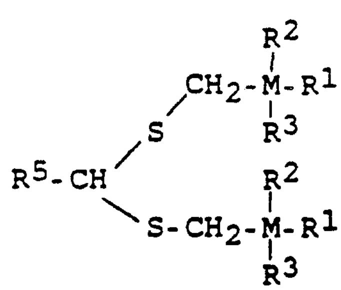
(XXVIII)

mit Chlor behandelt (siehe Methoden der org. Chemie (Houben-Weyl), Bd. 5/3, Georg Thieme Verlag, Stuttgart 1962, S. 756), wobei Verbindungen der allgemeinen Formel (VIII) bzw. (IX) mit Y = Cl entstehen.

In vergleichbarer Weise lassen sich Chlormethylsulfide der allgemeinen Formel (XXIX) erhalten

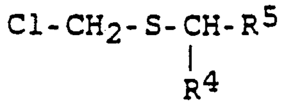

(XXIX)

Diese dienen als Edukte für die als Ausgangsverbindung beim Herstellungsverfahren d) benötigten Zwischenstufen der allgemeinen Formel (XIII), die sich daraus nach an sich literaturbekannten Verfahren herstellen lassen (siehe z.B. Methoden der org. Chemie (Houben-Weyl), Bd. 13/2a, Georg Thieme Verlag, Stuttgart 1973, S. 115).

Die als Ausgangsverbindungen beim Herstellungsverfahren e) zu verwendenden Silane der allgemeinen Formeln (XVII), (XVIII) und (XIX) lassen sich nach an sich literaturbekannten Verfahren herstellen (siehe Methoden der org. Chemie (Houben-Weyl), Bd. 13/5, Georg Thieme Verlag, Stuttgart 1980), indem man eine metallorganische Zwischenstufe der allgemeinen Formel (XIII) mit Silanen der Formeln (XXX), (XXXI) oder (XXXII)

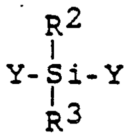

(XXX)

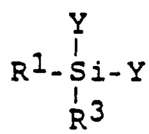

(XXXI)

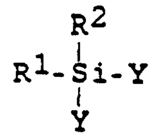

(XXXII)

umsetzt.

Die Darstellung der Zwischenstufen (IV), (V), (VI), (VII), (X), (XI), (XII), (XIV), (XV) und (XVI) ist entweder vorbeschrieben (EP-A 0249015 und DE-P 37 12 752.7) oder aber nach gängigen Literaturmethoden möglich (siehe u.a. die bisher zitierte Literatur).

Die genannten Verfahrensvarianten c), d) und e) werden bevorzugt in einem Verdünnungsmittel durchgeführt, dessen Natur von der Art der eingesetzten metallorg. Verbindung abhängt. Als Verdünnungsmittel eignen sich insbesondere aliphatische und aromatische Kohlenwasserstoffe wie z.B. Pentan, Hexan, Heptan, Cyclohexan, Petrolether, Benzin, Ligroin, Benzol, Toluol und Xylol, Ether wie z.B. Diethyl-und Dibutylether, Glykoldimethylether, Diglykoldimethylether, Tetrahydrofuran oder Dioxan und schließlich alle möglichen Gemische aus den zuvor genannten Lösungsmitteln.

Die Reaktionstemperatur liegt bei den obengenannten Verfahrensvarianten zwischen -110 °C und +150 °C, vorzugsweise zwischen -75 °C und +105 °C. Die Ausgangskomponenten werden gewöhnlich in äquimolaren Mengen eingesetzt. Ein Überschuß der einen oder anderen Reaktionskomponente ist jedoch möglich.

Für die weiter oben genannten Verfahrensvarianten a) und b) gilt im wesentlichen das gleiche wie für die Varianten c)-e). Es lassen sich jedoch noch weitere Verdünnungsmittel einsetzen. So eignen sich in diesen Fällen auch Alkohole wie z.B. Methanol, Ethanol, Propanol, i-Propanol oder Butanol, Amide, wie z.B. Dimethylformamid, Dimethylacetamid oder N-Methylpyrrolidon, Nitrile wie z.B. Acetonitril oder Butyronitril, Ketone wie z.B. Aceton oder MIBK, sowie Dimethylsulfoxid, Tetramethylensulfon oder Hexamethylphosphorsäuretriamid als Verdünnungsmittel. Die Verfahrensvarianten a) und b) lassen sich jedoch auch ohne Verdünnungsmittel durchführen.

Als Basen finden anorganische Basen wie z.B. Alkali- und Erdalkalimetall- hydroxide-, -hydride, -carbonate, -acetate oder -hydrogencarbonate, aber auch organische Basen wie z.B. Pyridin, Triethylamin, N,N-Diisopropyl-ethylamin oder Diazabicyclooctan Verwendung. Die Überführung der Mercaptane (II) bzw. (V) in ihre Salze (III) bzw. (VI) kann aber auch durch Zugabe von metallorganischen Reagenzien wie z.B. Methyllithium, Butyllithium, Methylmagnesiumhalogenid, Phenylmagnesiumhalogenid usw. bewerkstelligt werden.

Die Isolierung und gegebenenfalls Reinigung der Verbindungen der Formel (I) erfolgt nach allgemein üblichen Methoden, z.B. durch Abdampfen des Lösungsmittels (gegebenenfalls unter vermindertem Druck) und anschließendes Destillieren oder Chromatographieren oder durch Verteilen des Rohproduktes zwischen zwei Phasen und die sich daran anschließende übliche Aufarbeitung.

Die Verbindungen der allgemeinen Formel (I) sind in den meisten organischen Lösungsmitteln gut löslich.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta american, Leucophaea maderae, Blattella germanica, Acheta domesticus, Gryllotalpa spp., Locusta migratoria migratoriodes, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrate, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hypalopterus arundinis, Macrosiphum avenae, Myzus spp., Phorodon humuli, Rhopalosiphum padi, Empoasca spp., Euscelis bilobatus, Naphotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypielle, Bupalus piniarius, Cheimatobia brumata, Lithocolletis blancardella, Hyponomeuta padella, Plutella maculipennis, Malcosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia koehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus eleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Weiterhin haben die Verbindungen eine ausgezeichnete Wirkung gegen pflanzenschädigende Nematoden, beispielsweise solche der Gattungen Meloidogyne, Heterodera, Ditylenchus, Aphelenchoides, Radopholus, Globodera, Pratylenchus, Longidorus und Xiphinema.

Gegenstand der Erfindung sind auch Mittel, die die Verbindungen der Formel (I) neben geeigneten Formulierungshilfsmitteln enthalten.

Die erfindungsgemäßen Mittel enthalten die Wirkstoffe der Formel (I) im allgemeinen zu 1-95 Gew.-%. Sie können auf verschiedene Art formuliert werden, je nachdem wie es durch die biologischen und/oder chemischphysikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc. , N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohle, Alkyl- oder Alkylphenol-sulfonate und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleoylmethyltaurinsaures Natrium enthalten. Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid- Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes aus adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus

diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a.
Bevorzugte Mischungspartner sind

**1. aus der Gruppe der Phosphorverbindungen**

Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulffon, Dialifos, Diazinon, Dichlorvos, Dicrotophos, 0,0-1,2,2,2-tetrachlorethylphosphorthioate (SD 208 304), Dimethoate, Disulfoton, EPN, Ethion, Ethoprophos, Etrimfos, Famphur, Fenamiphos, Fenitrothion, Fensulfothion, Fenthion, Fonofos, Formothion, Heptenophos, Isazophos, Isothioate, Isoxathion, Malathion, Methacrifos, Methamidophos, Methidathion, Salithion, Mevinphos, Monocrotophos, Naled, Omethoate, Oxydemetonmethyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos Terbufos, Tetrachlorvinphos, Thiometon, Triazophos, Trichlorphon, Vamidothion;

**2. aus der Gruppe der Carbamate**

Aldicarb, 2-sec.-Butylphenylmethycarbamate (BPMC), Carbaryl, Carbofuran, Carbosulfan, Cloethocarb, Benfuracarb, Ethiofencarb, Furathiocarb, Isoprocarb, Methomyl, 5-Methyl-m- cumenylbutyryl(methyl)-carbamate, Oxamyl, Pirimicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-methylthio(ethylideneamino)-N-methyl-N-(morpholinothio)-carbamate (UC 51717);

**3. aus der Gruppe der Carbonsäureester**

Allethrin, Alphametrin, 5-Benzyl-3-furylmethyl-(E)-(1R)-cis-2,2-di-methyl-3-(2-oxothiolan-3-ylidenemethyl) cyclopropanecarboxylate, Bioallethrin, Bioallethrin((S)-cyclopentylisomer), Bioresmethrin, Biphenate, (RS)-1-Cyano-1-(6-phenoxy-2-pyridyl)-methyl-(1RS)-trans-3-(4-tert. butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Phenothrin ((R)-Isomer), d-Pralethrin, Pyrethrine (natürliche Produkte), Resmethrin, Tefluthrin, Tetramethrin, Tralomethrin, Hydroprene, Methoprene, Kinoprene;

**4. aus der Gruppe der Amidine**

Amitraz, Chlordimeform;

**5. aus der Gruppe der Zinnverbindungen**

Cyhexatin, Fenbutatinoxide, Azocyclotin;

**6. Sonstige**

Abamectin, Bacilllus thuringiensis, Bensultap, Binapacryl, Bromopropylate, Buprofezin, Camphechlor, Cartap, Chlorobenzilate, Chlorfluazuron, 2-(4-(Chlorphenyl)-4,5-di-phenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Dichlor-4-(1,1,2,2-tetrafluorethoxy)phenylamino) carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl)(dimethyl)(3-(3-phenoxyphenyl)propyl)silan, (4-Ethoxyphenyl)(3-(4-fluoro-3-phenoxyphenyl)-propyl) dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron,O-Ethyl-N-[2-(4-phenoxy)phenoxyethyl]carbamat, O-[2-(4-Phenoxy) phenoxyethyl]propionaldoxim, 1-(4-Phenoxy-phenoxy)-2-(2-pyridinoxy)propan.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,00001 und 1 Gew.-% liegen. Die Anwendung geschieht in einer den Anwendungsformen angepaßten üblichen Weise.

Die erfindungsgemäßen Wirkstoffe eignen sich auch zur Bekämpfung von Ekto- und Endoparasiten vorzugsweise von ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch orale Anwendung in Form von beispielsweise Tabletten, Kapseln, Tränken, Granulaten, druch dermale Anwendung in Form beispielsweise des Tauchens (Dippen), Sprühens (Sprayen), Aufgießens (pour-on and spoton) und des Einpuderns.

Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen.

Nachfolgende Beispiele dienen zur Erläuterung der Erfindung.

## A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

## B. Chemische Beispiele

### Herstellungsvorschriften

1) Eine Mischung aus 25,6 g (100 mMol) 5-Brom-2-(2,2,2-trifluorethoxy)pyridin (aus 2,5-Dibrompyridin und 2,2,2-Trifluorethanol/Natriumhydrid in DMSO), 20 g (140 mMol) Chlor-chlormethyl-dimethylsilan und 120 ml wasserfreiem Tetrahydrofuran wird bei -70° C tropfenweise mit 56 ml (≙ 140 mMol) einer 2,5 M-n-Butyllithiumlösung in Hexan versetzt. Man rührt nach beendeter Zugabe weitere 30 min. bei -70° C, läßt die Reaktionsmischung langsam Raumtemp. annehmen und versetzt schließlich mit 5 ml Wasser. Nach Eindampfen der Reaktionslösung wird der Rückstand in 200 ml Hexan aufgenommen und zweimal mit Wasser gewaschen. Die über $Na_2SO_4$ getrocknete Hexanphase enthält neben dem gewünschten Zwischenprodukt -< 2-(2,2,2-Trifluorethoxy)-pyrid-5-yl)-(dimethyl)(chlormethyl)-silan- als Nebenprodukt Butyl-chlormethyldimethylsilan.

Letzteres wird durch Andestillieren des Rohproduktes bis 50 °C (Innentemp.) bei 0,2 mbar weitestgehend entfernt. Das im Rückstand verbleibende Silylpyridin wird ohne weitere Reinigung bei Raumtemp. zu einer Lösung von 15 g (130 mMol) Kaliumthioacetat in 100 ml wasserfreiem DMSO getropft. Nach 1,5 h bei 60-70 °C gießt man die Reaktionsmischung auf 1 l Eiswasser und extrahiert das Produkt mit mehreren Etherportionen. Die Extrakte werden dreimal mit 20 %iger Kochsalzlösung gewaschen, über $Na_2SO_4$ getrocknet und eingedampft. Die abschließende Vakuumdestillation liefert 22,3 g (69 %) < 2-(2,2,2-Trifluorethoxy)-pyrid-5-yl>-(dimethyl)-(acetylthiomethyl)silan als blaßgelbes Öl vom Kp. 95-100 °C/0,1 mbar.

2) Zu einer Suspension von 5,3 g (140 mMol) Lithiumaluminiumhydrid in 50 ml wasserfreiem Ether tropft man unter Rühren bei Raumtemperatur die Lösung von 22,3 g (69 mMol) <2-(2,2,2-Trifluorethoxy)-pyrid-5-yl>-(dimethyl)-(acetylthiomethyl)-silan in 200 ml wasserfreiem Ether. Nach weiteren 3 h wird das Reaktionsgemisch durch tropfenweise Zugabe von wenig Wasser zersetzt. Der dabei sich bildende, feste Niederschlag wird abgesaugt und gründlich mit Ether gewaschen. Die Filtrate liefern nach Eindampfen 8,6 g (44 %) (Mercaptomethyl)-(dimethyl)-<2-(2,2,2-trifluorethoxy)-pyrid-5-yl)-silan als blaßgelbes Öl.

3) Zu einer Lösung von 5,3 g (18,8 mMol) 4-Fluoro-3-phenoxybenzylbromid in 10 ml wasserfreiem THF tropft man bei -60 °C zunächst die Lösung von 4,3 g (15,3 mMol) (Mercaptomethyl)-(dimethyl)-<2-(2,2,2-trifluorethoxy)pyrid-5-yl>silan in 10 ml THF, dann 10 ml 1,6 M-Methyllithiumlösung in Ether. Man läßt die Reaktionslösung anschließend über einen Zeitraum von ca. 2 h Raumtemp. annehmen und rührt weitere 2 h bei 20-25 °C. Nach Zugabe von 200 ml Wasser wird das Produkt mit zwei 100 ml-Portionen Hexan extrahiert. Die Extrakte liefern nach zweimaligem Waschen mit Wasser, Trocknen ($Na_2SO_4$) und Eindampfen 8,1 g Rohprodukt. Bei der abschließenden Chromatographie an Kieselgel mit Methylenchlorid/Hexan (1:1) erhält man 3,1 g (42 %) (Dimethyl)-<2- (2,2,2-trifluorethoxy)-pyrid-5-yl>-<(4-fluoro-3-phenoxy-benzyl)-thiomethyl>silan (Bsp. 26/Tab. 1) als blaßgelbes Öl vom $Kp_{0,05}$ = 230-240 °C (Kugelrohr).

In Anlehnung an diese Vorschriften lassen sich die nachfolgend aufgeführten Verbindungen mit M = Si darstellen. Auch die Synthesen der nachfolgend aufgeführten Verbindungen mit M = C verlaufen in ihren Teilschritten 2) und 3) völlig analog. Die Darstellung der dem Produkt aus Teilschritt 1) analogen Kohlenstoffverbindungen ist in DE-P 37 12 752.7 beschrieben.

Tabelle 1:

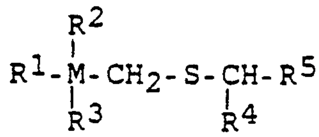

(I)

$M = Si,\ R^2 = R^3 = CH_3$

| Nr. | $R^1$ | $R^4$ | $R^5$ | Physikalische Daten |
|---|---|---|---|---|
| 1 | 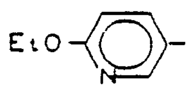 | H | 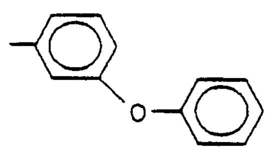 | $Kp_{0.1}$ = 240°C |
| 2 | " | H | 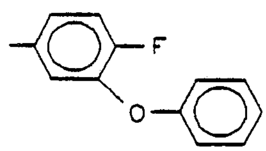 | $Kp_{0.03}$ = 235-240°C |
| 3 | " | H | 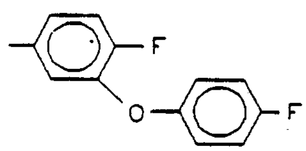 | |
| 4 | " | H | 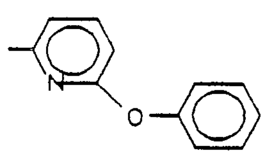 | $Kp_{0.2}$ = 245-250°C |
| 5 | " | H | 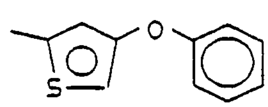 | $Kp_{0.1}$ = 235-245°C |
| 6 | | CN | 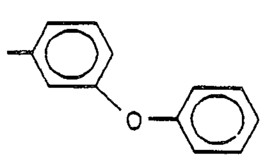 | hochviskoses Öl |

Fortsetzung der Tabelle 1:

| Nr. | $R^1$ | $R^4$ | $R^5$ | Physikalische Daten |
|---|---|---|---|---|
| 7 | 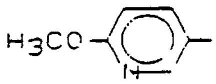 | H | 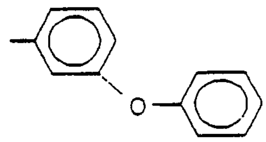 | |
| 8 | | H | 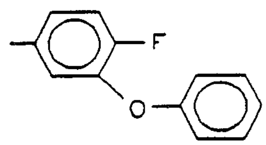 | $Kp_{0.2}$ = 240-245°C |
| 9 | " | H | 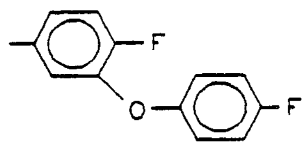 | |
| 10 | " | H | 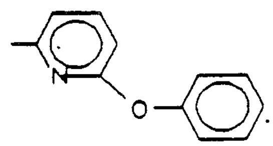 | |
| 11 | " | H | 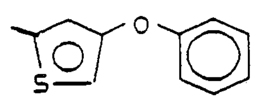 | $Kp_{0.05}$ = 235-245°C |
| 12 | " | CN | 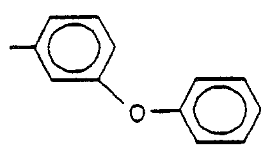 | |
| 13 | 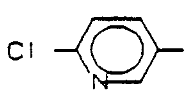 | H | 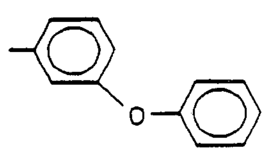 | |
| 14 | " | H | 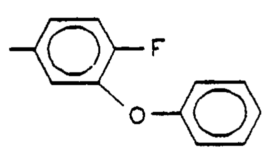 | $Kp_{0.01}$ = 220-225°C |

Fortsetzung der Tabelle 1:

| Nr. | $R^1$ | $R^4$ | $R^5$ | Physikalische Daten |
|---|---|---|---|---|
| 15 | " | H | 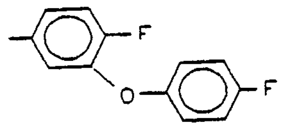 | |
| 16 | " | H | 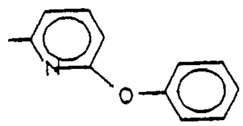 | |
| 17 | " | H | 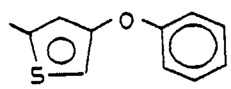 | $Kp_{0.07}$ = 230°C |
| 18 | " | CN | 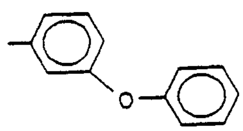 | |
| 19 | 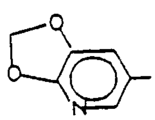 | H | 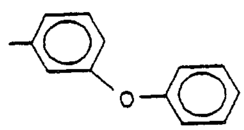 | $Kp_{0.1}$ = 230-235°C |
| 20 | " | H | 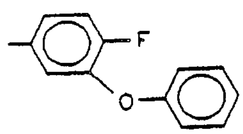 | $Kp_{0.05}$ = 230-240°C |
| 21 | " | H | 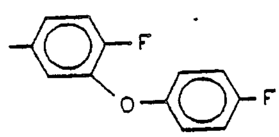 | |
| 22 | " | H | 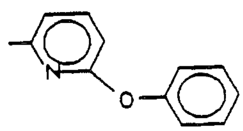 | $Kp_{0.02}$ = 240-250°C |

Fortsetzung der Tabelle 1:

| Nr. | $R^1$ | $R^4$ | $R^5$ | Physikalische Daten |
|---|---|---|---|---|
| 23 | " | H | 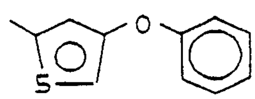 | |
| 24 | " | CN | 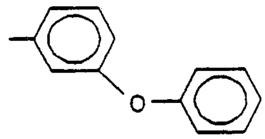 | |
| 25 | $F_3C-CH_2-O-$ 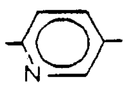 | H | 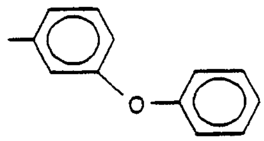 | $Kp_{0.2}$ = 245°C |
| 26 | " | H | 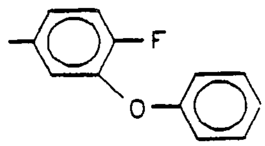 | $Kp_{0.05}$ = 230-240°C |
| 27 | " | H | 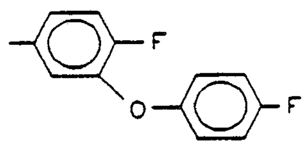 | |
| 28 | " | H | 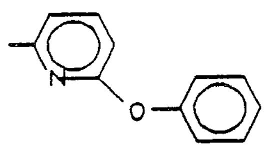 | |
| 29 | " | H | 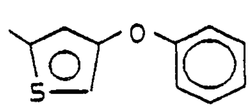 | $Kp_{0.05}$ = 230-240°C |
| 30 | " | CN | 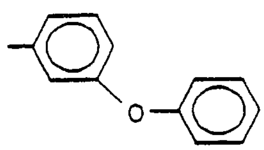 | |

Fortsetzung der Tabelle 1:

| Nr. | $R^1$ | $R^4$ | $R^5$ | Physikalische Daten |
|---|---|---|---|---|
| 31 | 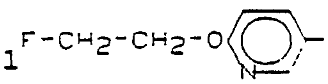 | H | 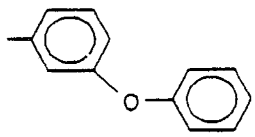 | |
| 32 | " | H | 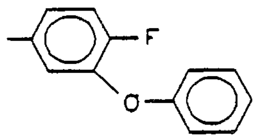 | $Kp_{0.07}$ = 235°C |
| 33 | " | H | 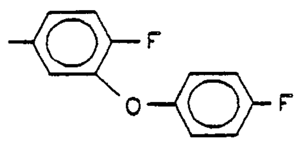 | |
| 34 | " | H | 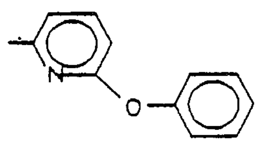 | |
| 35 | " | H | 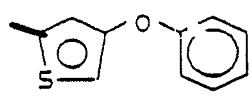 | |
| 36 | " | CN | 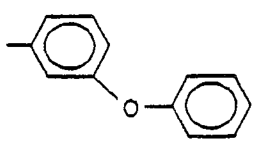 | |
| 37 | 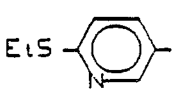 | H | 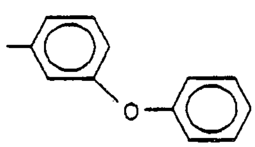 | $Kp_{0.05}$ = 240-245°C |
| 38 | " | H | 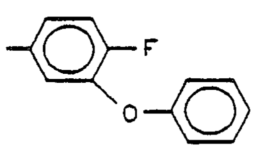 | $Kp_{0.01}$ = 235-240°C |

**Fortsetzung der Tabelle 1:**

| Nr. | $R^1$ | $R^4$ | $R^5$ | Physikalische Daten |
|---|---|---|---|---|
| 39 | " | H | 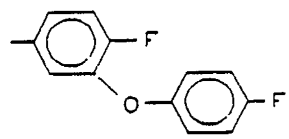 | |
| 40 | " | H | 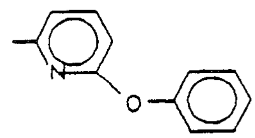 | |
| 41 | " | H | 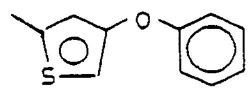 | |
| 42 | " | CN | 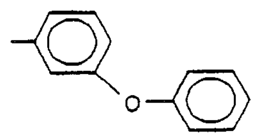 | |
| 43 | 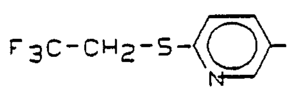 | H | 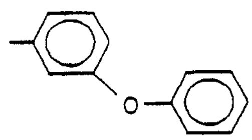 | |
| 44 | " | H | 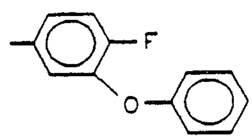 | |
| 45 | " | H | 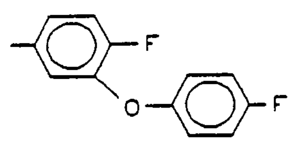 | |

Fortsetzung der Tabelle 1:

| Nr. | $R^1$ | $R^4$ | $R^5$ | Physikalische Daten |
|---|---|---|---|---|
| 46 | " | H | 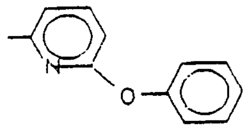 | |
| 47 | " | H | 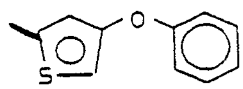 | |
| 48 | " | CN | 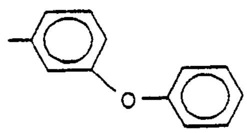 | |
| 49 | 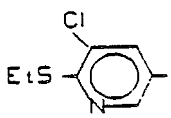 | H | 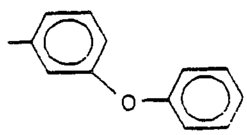 | |
| 50 | " | H | 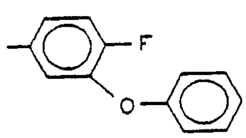 | $Kp_{0.05}$ >250°C |
| 51 | " | H | 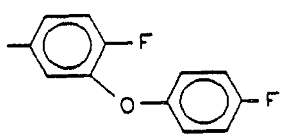 | |
| 52 | " | H | 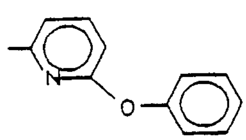 | |

Fortsetzung der Tabelle 1:

| Nr. | $R^1$ | $R^4$ | $R^5$ | Physikalische Daten |
|---|---|---|---|---|
| 53 | " | H | 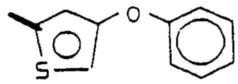 | |
| 54 | " | CN | 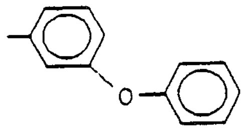 | |
| 55 | 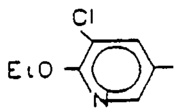 | H | 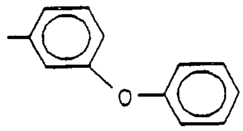 | $Kp_{0.01}$ = 250°C |
| 56 | " | H | 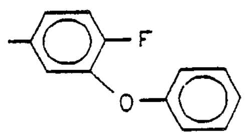 | $Kp_{0.01}$ = 250°C |
| 57 | " | H | 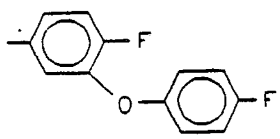 | |
| 58 | " | H | 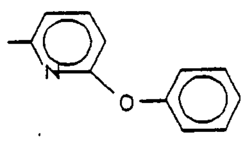 | |
| 59 | " | H | 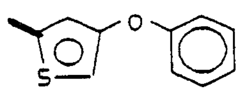 | |

**Fortsetzung der Tabelle 1:**

| Nr. | $R^1$ | $R^4$ | $R^5$ | Physikalische Daten |
|---|---|---|---|---|
| 60 | " | CN | 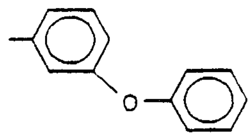 | |
| 61 | 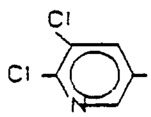 | H | 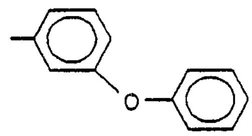 | $Kp_{0.05}$ = 240-245°C |
| 62 | " | H | 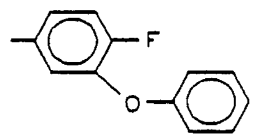 | $Kp_{0.03}$ = 240-245°C |
| 63 | " | H | 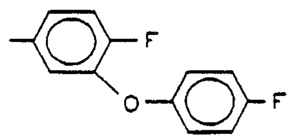 | $Kp_{0.05}$ = 240-245°C |
| 64 | " | H | 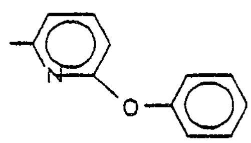 | |
| 65 | " | H | 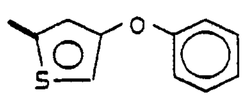 | $Kp_{0.05}$ = 240°C |
| 66 | " | CN | 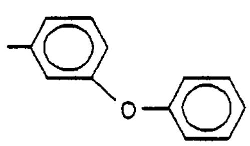 | |

Fortsetzung der Tabelle 1:

| Nr. | $R^1$ | $R^4$ | $R^5$ | Physikalische Daten |
|---|---|---|---|---|
| 67 | 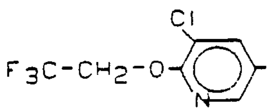 | H | 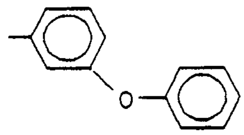 | $Kp_{0.05}$ = 250°C |
| 68 | " | H | 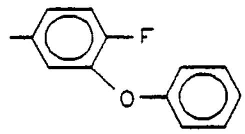 | $Kp_{0.02}$ = 250°C |
| 69 | " | H | 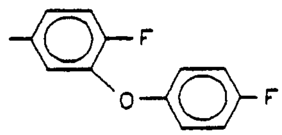 | |
| 70 | " | H | 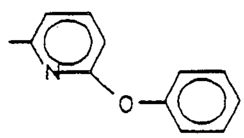 | |
| 71 | " | H | 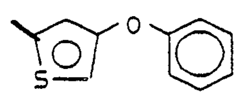 | |
| 72 | " | CN | 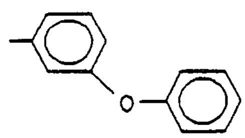 | |
| 73 | 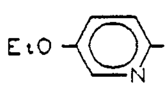 | H | 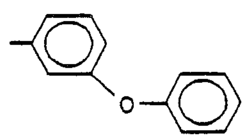 | |

**Fortsetzung der Tabelle 1:**

| Nr. | $R^1$ | $R^4$ | $R^5$ | Physikalische Daten |
|---|---|---|---|---|
| 74 | " | H | 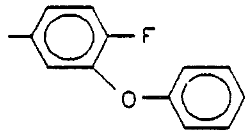 | $Kp_{0.01}$ = 240-245°C |
| 75 | " | H | 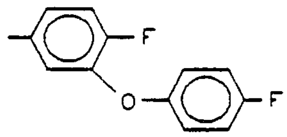 | |
| 76 | " | H | 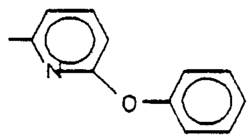 | |
| 77 | " | H | 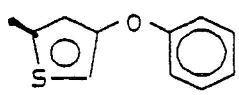 | |
| 78 | " | CN | 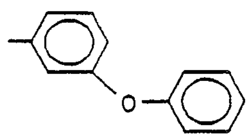 | |
| 79 | 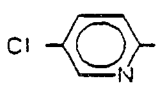 | H | 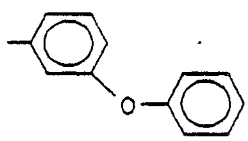 | |
| 80 | " | H | 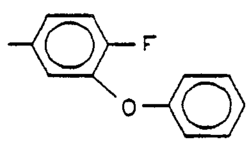 | $Kp_{0.05}$ = 230-235°C |

Fortsetzung der Tabelle 1:

| Nr. | $R^1$ | $R^4$ | $R^5$ | Physikalische Daten |
|---|---|---|---|---|
| 81 | " | H | 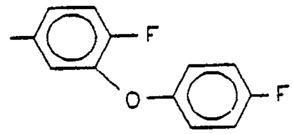 | |
| 82 | " | H | 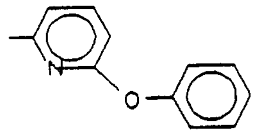 | |
| 83 | " | H | 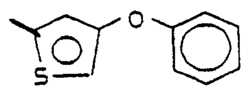 | |
| 84 | " | CN | 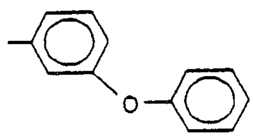 | |
| 85 | 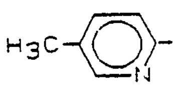 | H | 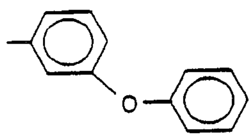 | $Kp_{0.02}$ = 240°C |
| 86 | " | H | 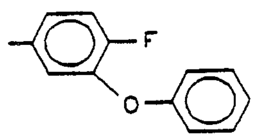 | |
| 87 | " | H | 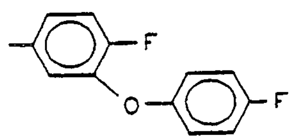 | |

Fortsetzung der Tabelle 1:

| Nr. | $R^1$ | $R^4$ | $R^5$ | Physikalische Daten |
|---|---|---|---|---|
| 88 | " | H | 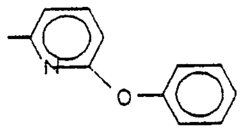 | |
| 89 | " | H | 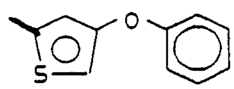 | |
| 90 | " | CN | 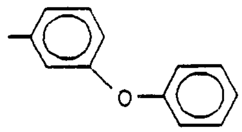 | |
| 91 | 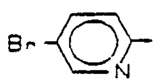 | H | 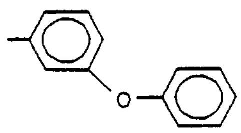 | $Kp_{0.02}$ = 235-240°C |
| 92 | " | H | 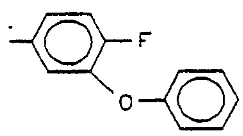 | |
| 93 | " | H | 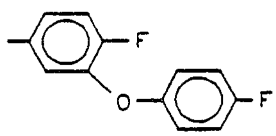 | |
| 94 | " | H | 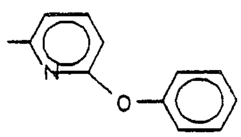 | |

Fortsetzung der Tabelle 1:

| Nr. | $R^1$ | $R^4$ | $R^5$ | Physikalische Daten |
|---|---|---|---|---|
| 95 | " | H | 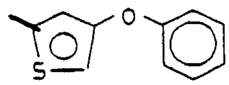 | |
| 96 | " | CN | 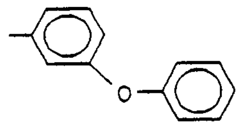 | |
| 97 | 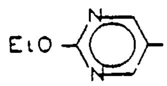 | H | 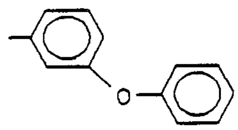 | blaßgelbes Öl $R_f(CH_2Cl_2/$ Heptan 4:1) = 0.25 |
| 98 | " | H | 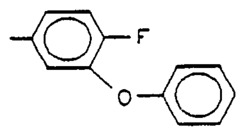 | blaßgelbes Öl $R_f(CH_2Cl_2/$ Heptan 4:1) = 0.25 |
| 99 | " | H | 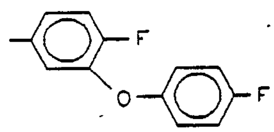 | |
| 100 | " | H | 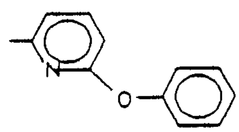 | |
| 101 | " | H | 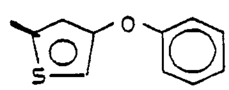 | |

**Fortsetzung der Tabelle 1:**

| Nr. | $R^1$ | $R^4$ | $R^5$ | Physikalische Daten |
|---|---|---|---|---|
| 102 | " | CN | 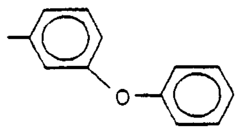 | |
| 103 | 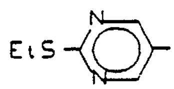 | H | 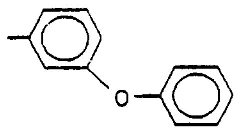 | |
| 104 | " | H | 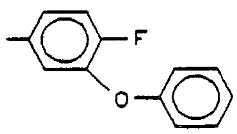 | |
| 105 | " | H | 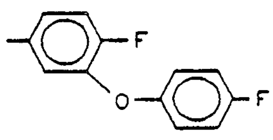 | |
| 106 | " | H | 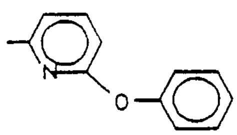 | |
| 107 | " | H | 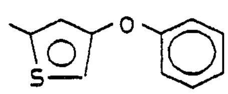 | |
| 108 | " | CN | 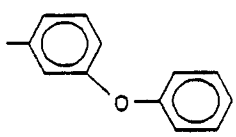 | |

**Fortsetzung der Tabelle 1:**

| Nr. | $R^1$ | $R^4$ | $R^5$ | Physikalische Daten |
|---|---|---|---|---|
| 109 | 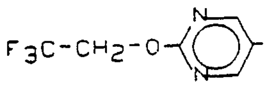 | H | 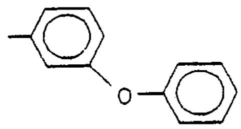 | blaßgelbes Öl $R_f(CH_2Cl_2)=0.3$ |
| 110 | " | H | 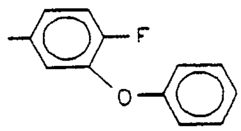 | blaßgelbes Öl $R_f(CH_2Cl_2)=0.3$ |
| 111 | " | H | 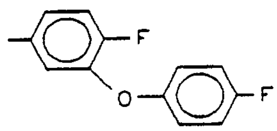 | |
| 112 | " | H | 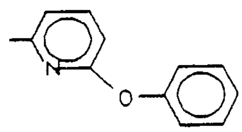 | |
| 113 | " | H | 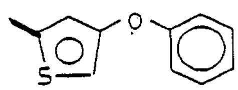 | |
| 114 | " | CN | 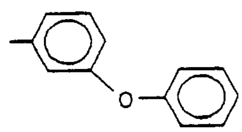 | |
| 115 | 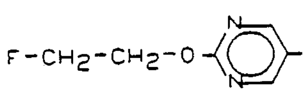 | H | 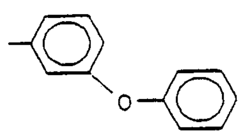 | |

Fortsetzung der Tabelle 1:

| Nr. | $R^1$ | $R^4$ | $R^5$ | Physikalische Daten |
|---|---|---|---|---|
| 116 | " | H | 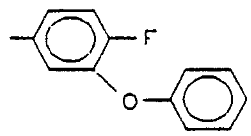 | |
| 117 | " | H | 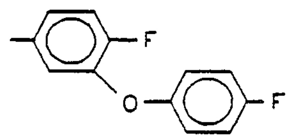 | |
| 118 | " | H | 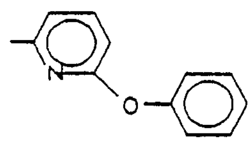 | |
| 119 | " | H | 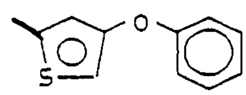 | |
| 120 | " | CN | 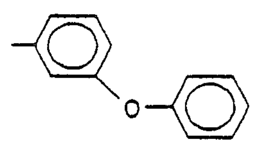 | |
| 121 | 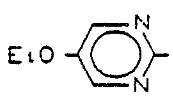 | H | 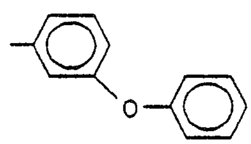 | |
| 122 | " | H | 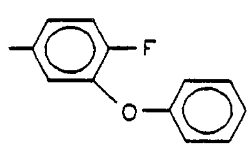 | |

Fortsetzung der Tabelle 1:

| Nr. | $R^1$ | $R^4$ | $R^5$ | Physikalische Daten |
|---|---|---|---|---|
| 123 | " | H | 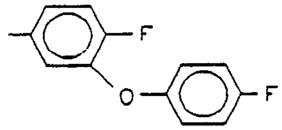 | |
| 124 | " | H | 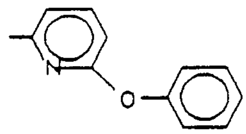 | |
| 125 | " | H | 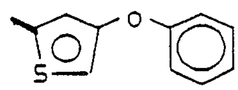 | |
| 126 | " | CN | 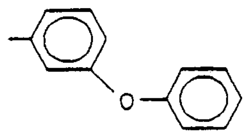 | |
| 127 | 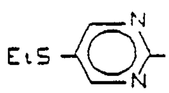 | H | 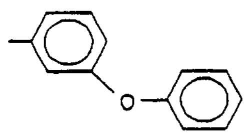 | |
| 128 | " | H | 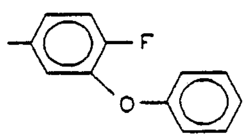 | |
| 129 | " | H | 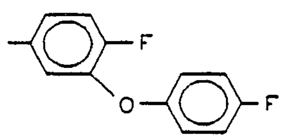 | |

Fortsetzung der Tabelle 1:

| Nr. | $R^1$ | $R^4$ | $R^5$ | Physikalische Daten |
|---|---|---|---|---|
| 130 | " | H | 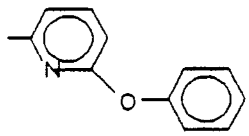 | |
| 131 | " | H | 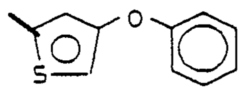 | |
| 132 | " | CN | 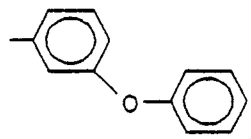 | |

**Tabelle 2:** M = C; $R^2 = R^3 = CH_3$

| Nr. | $R^1$ | $R^4$ | $R^5$ | Physikalische Daten |
|---|---|---|---|---|
| 133 | 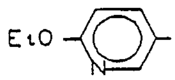 | H | 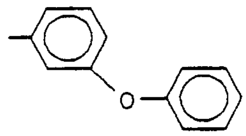 | $Kp_{0.001}$ = 224-230°C |
| 134 | " | H | 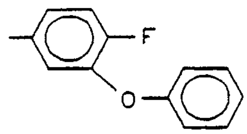 | $Kp_{0.05}$ = 230-235°C |
| 135 | " | H | 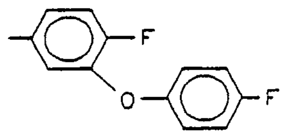 | |
| 136 | " | H | 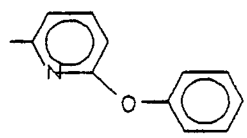 | $Kp_{0.0005}$ = 210°C |
| 137 | " | H | 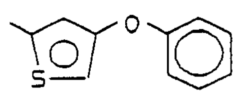 | $Kp_{0.01}$ = 220-230°C |
| 138 | " | CN | 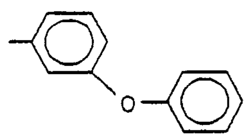 | |
| 139 | 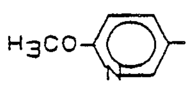 | H | 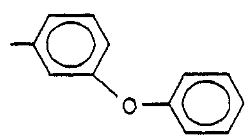 | |

**Fortsetzung von Tabelle 2:**

| Nr. | $R^1$ | $R^4$ | $R^5$ | Physikalische Daten |
|---|---|---|---|---|
| 140 | " | H | 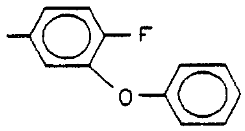 | |
| 141 | " | H | 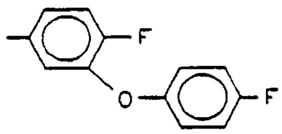 | |
| 142 | " | H | 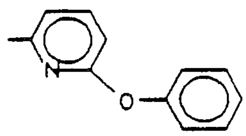 | |
| 143 | " | H | 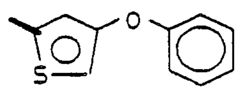 | |
| 144 | " | CN | 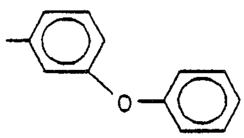 | |
| 145 | 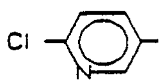 | H | 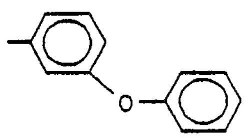 | |
| 146 | " | H | 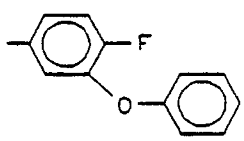 | |

**Fortsetzung von Tabelle 2:**

| Nr. | $R^1$ | $R^4$ | $R^5$ | Physikalische Daten |
|---|---|---|---|---|
| 147 | " | H | 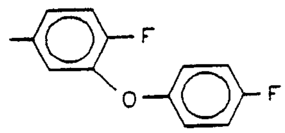 | |
| 148 | " | H | 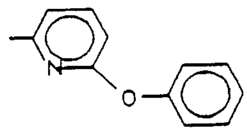 | |
| 149 | " | H | 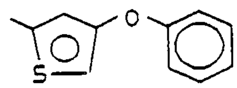 | |
| 150 | " | CN | 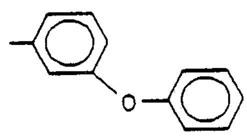 | |
| 151 | 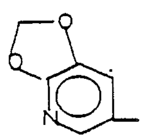 | H | 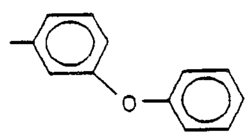 | |
| 152 | " | H | 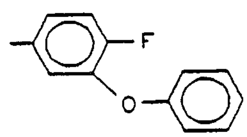 | |
| 153 | " | H | 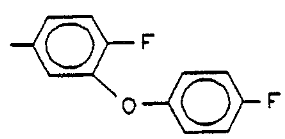 | |

Fortsetzung von Tabelle 2:

| Nr. | $R^1$ | $R^4$ | $R^5$ | Physikalische Daten |
|---|---|---|---|---|
| 154 | " | H | 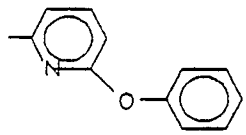 | |
| 155 | " | H | 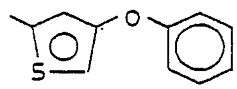 | |
| 156 | " | CN | 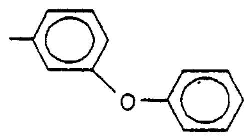 | |
| 157 | 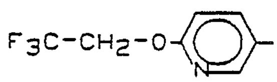 | H | 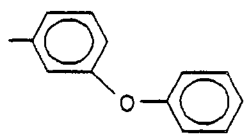 | $Kp_{0.001}$ = 225-235°C |
| 158 | " | H | 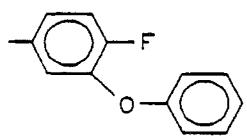 | $Kp_{0.01}$ = 220-230°C |
| 159 | " | H | 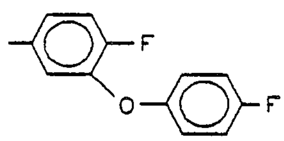 | $Kp_{0.01}$ = 230-235°C |
| 160 | " | H | 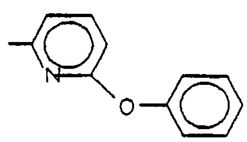 | |

Fortsetzung von Tabelle 2:

| Nr. | $R^1$ | $R^4$ | $R^5$ | Physikalische Daten |
|---|---|---|---|---|
| 161 | " | H | 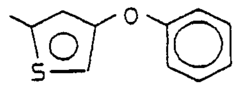 | |
| 162 | " | CN | 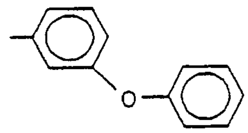 | |
| 163 | 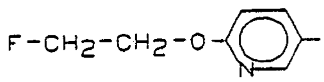 | H | 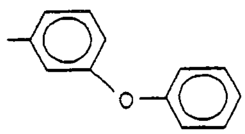 | |
| 164 | " | H | 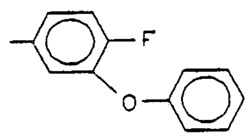 | $Kp_{0.001}$ = 235°C |
| 165 | " | H | 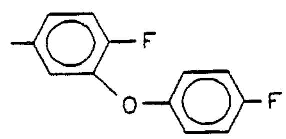 | |
| 166 | " | H | 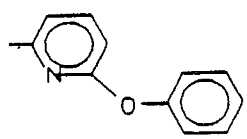 | |
| 167 | " | H | 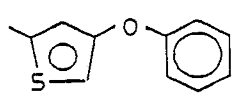 | |

**Fortsetzung von Tabelle 2:**

| Nr. | $R^1$ | $R^4$ | $R^5$ | Physikalische Daten |
|---|---|---|---|---|
| 168 | " | CN | 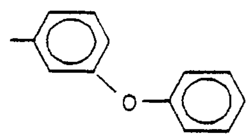 | |
| 169 | 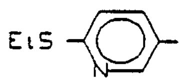 | H | 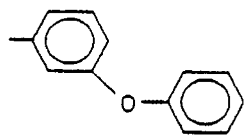 | |
| 170 | " | H | 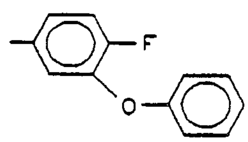 | |
| 171 | " | H | 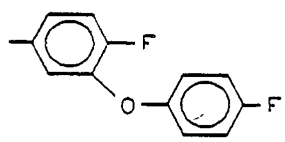 | |
| 172 | " | H | 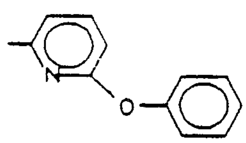 | |
| 173 | " | H | 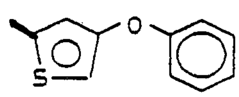 | |
| 174 | " | CN | 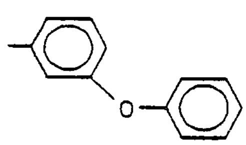 | |

Fortsetzung von Tabelle 2:

| Nr. | $R^1$ | $R^4$ | $R^5$ | Physikalische Daten |
|---|---|---|---|---|
| 175 | 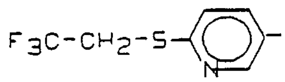 | H | 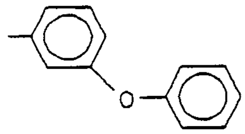 | |
| 176 | " | H | 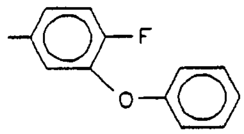 | |
| 177 | " | H | 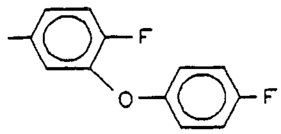 | |
| 178 | " | H | 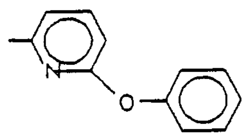 | |
| 179 | " | H | 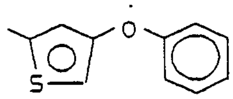 | |
| 180 | " | CN | 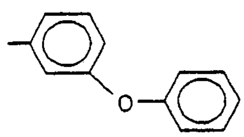 | |
| 181 | 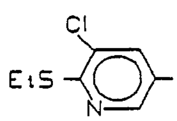 | H | 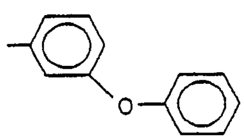 | $Kp_{0.001}$ = 230-240°C |

Fortsetzung von Tabelle 2:

| Nr. | $R^1$ | $R^4$ | $R^5$ | Physikalische Daten |
|---|---|---|---|---|
| 182 | " | H | 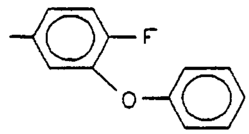 | $Kp_{0.001}$ = 240°C |
| 183 | " | H | 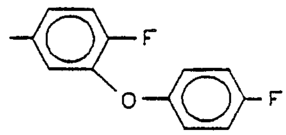 | |
| 184 | " | H | 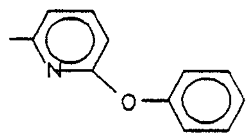 | |
| 185 | " | H | 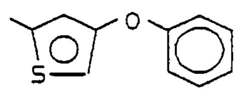 | |
| 186 | " | CN | 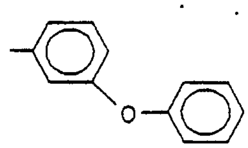 | |
| 187 | 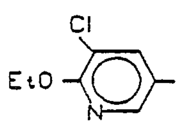 | H | 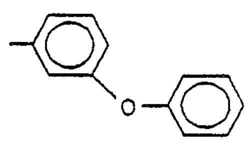 | $Kp_{0.001}$ = 235-240°C |
| 188 | " | H | 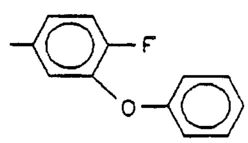 | $Kp_{0.003}$ = 240°C |

Fortsetzung von Tabelle 2:

| Nr. | $R^1$ | $R^4$ | $R^5$ | Physikalische Daten |
|---|---|---|---|---|
| 189 | " | H | 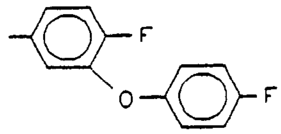 | |
| 190 | " | H | 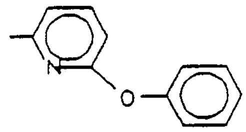 | |
| 191 | " | H | 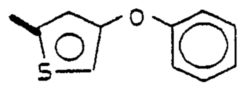 | |
| 192 | " | CN | 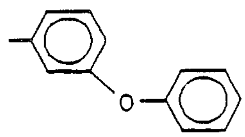 | |
| 193 | 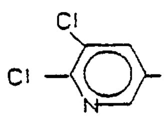 | H | 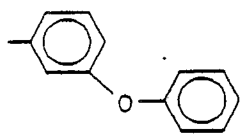 | |
| 194 | " | H | 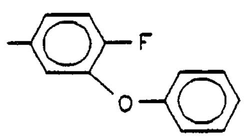 | |
| 195 | " | H | 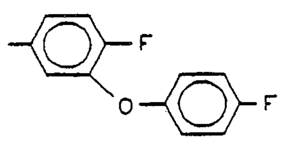 | |

Fortsetzung von Tabelle 2:

| Nr. | $R^1$ | $R^4$ | $R^5$ | Physikalische Daten |
|---|---|---|---|---|
| 196 | " | H | 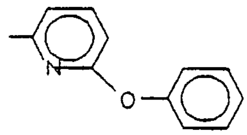 | |
| 197 | " | H | 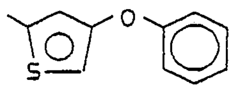 | |
| 198 | " | CN | 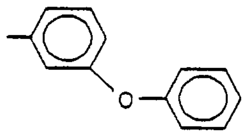 | |
| 199 | 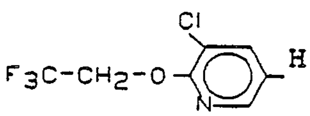 | H | 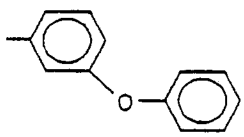 | $Kp_{0.001}$ = 220-225°C |
| 200 | " | H | 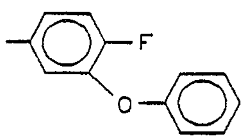 | $Kp_{0.003}$ = 230°C |
| 201 | " | H | 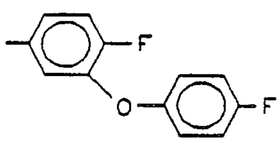 | |
| 202 | " | H | 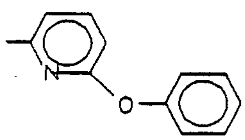 | |

Fortsetzung von Tabelle 2:

| Nr. | $R^1$ | $R^4$ | $R^5$ | Physikalische Daten |
|---|---|---|---|---|
| 203 | " | H | 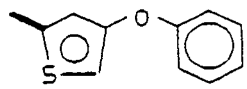 | |
| 204 | " | CN | 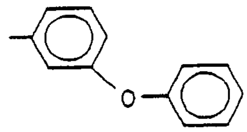 | |
| 205 | 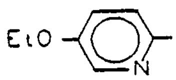 | | 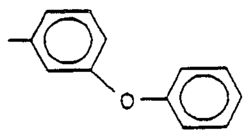 | |
| 206 | " | H | 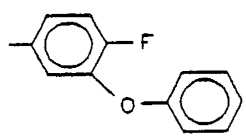 | |
| 207 | " | H | 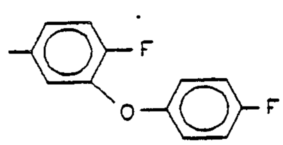 | |
| 208 | " | H | 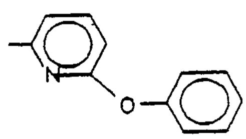 | |
| 209 | " | H | 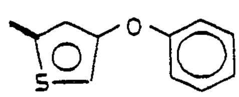 | |

Fortsetzung von Tabelle 2:

| Nr. | $R^1$ | $R^4$ | $R^5$ | Physikalische Daten |
|---|---|---|---|---|
| 210 | " | CN | 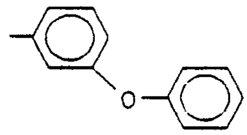 | |
| 211 | 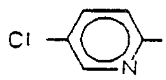 | H | 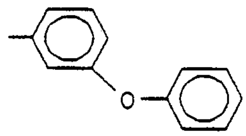 | |
| 212 | " | H | 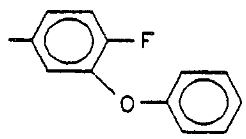 | |
| 213 | " | H | 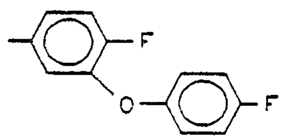 | |
| 214 | " | H | 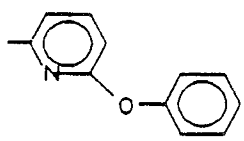 | |
| 215 | " | H | 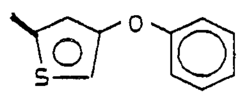 | |
| 216 | " | CN | 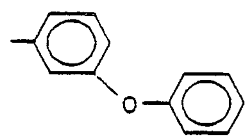 | |

Fortsetzung von Tabelle 2:

| Nr. | $R^1$ | $R^4$ | $R^5$ | Physikalische Daten |
|---|---|---|---|---|
| 217 | 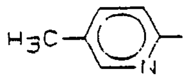 | H | 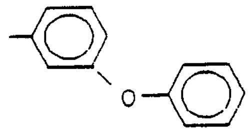 | |
| 218 | " | H | 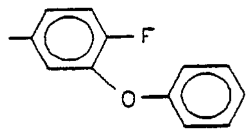 | |
| 219 | " | H | 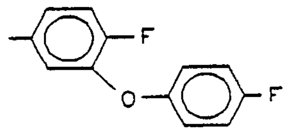 | |
| 220 | " | H | 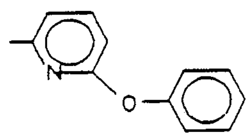 | |
| 221 | " | H | 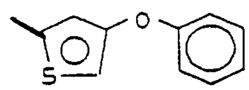 | |
| 222 | " | CN | 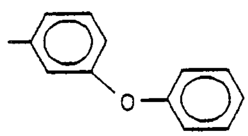 | |
| 223 | 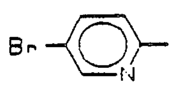 | H | 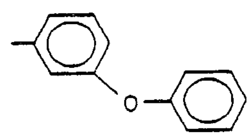 | |

Fortsetzung von Tabelle 2:

| Nr. | $R^1$ | $R^4$ | $R^5$ | Physikalische Daten |
|---|---|---|---|---|
| 224 | " | H | 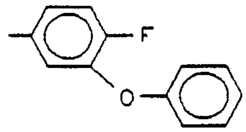 | |
| 225 | " | H | 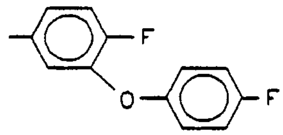 | |
| 226 | " | H | 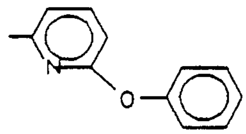 | |
| 227 | " | H | 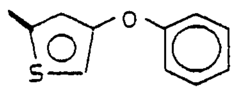 | |
| 228 | " | CN | 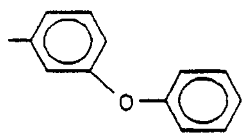 | |
| 229 | 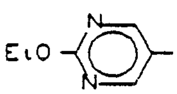 | | 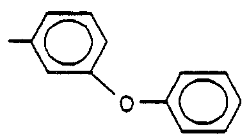 | |
| 230 | " | H | 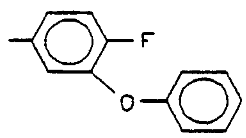 | |

**Fortsetzung von Tabelle 2:**

| Nr. | $R^1$ | $R^4$ | $R^5$ | Physikalische Daten |
|---|---|---|---|---|
| 231 | " | H | 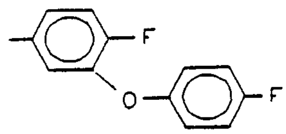 | |
| 232 | " | H | 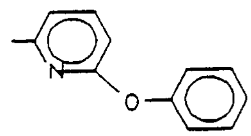 | |
| 233 | " | H | 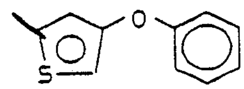 | |
| 234 | " | CN | 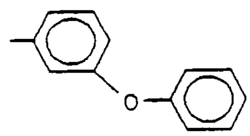 | |
| 235 | 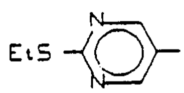 | H | 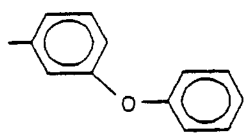 | |
| 236 | " | H | 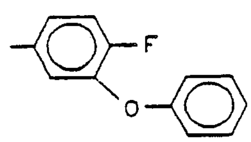 | |
| 237 | " | H | 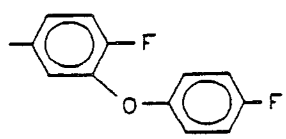 | |

Fortsetzung von Tabelle 2:

| Nr. | $R^1$ | $R^4$ | $R^5$ | Physikalische Daten |
|---|---|---|---|---|
| 238 | " | H | 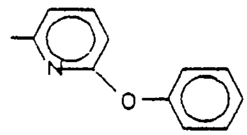 | |
| 239 | " | H | 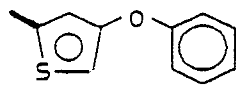 | |
| 240 | " | CN | 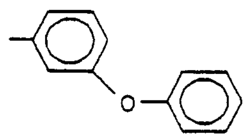 | |
| 241 | 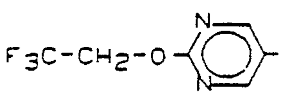 | | 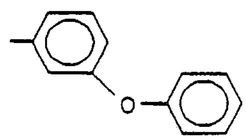 | |
| 242 | " | H | 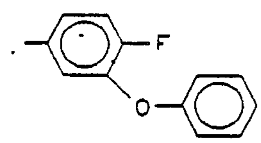 | |
| 243 | " | H | 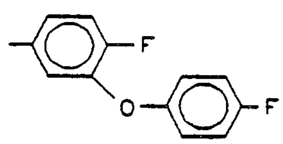 | |
| 244 | " | H | 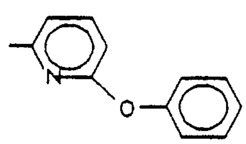 | |

Fortsetzung von Tabelle 2:

| Nr. | $R^1$ | $R^4$ | $R^5$ | Physikalische Daten |
|---|---|---|---|---|
| 245 | " | H | 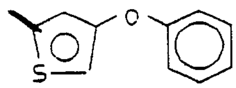 | |
| 246 | " | CN | 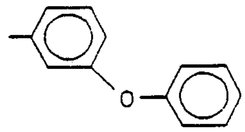 | |
| 247 | $F-CH_2-CH_2-O-$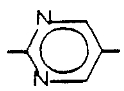 | H | 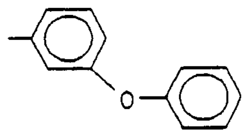 | |
| 248 | " | H | 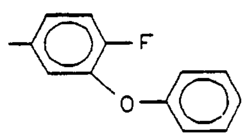 | |
| 249 | " | H | 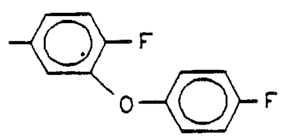 | |
| 250 | " | H | 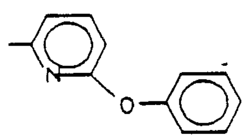 | |
| 251 | " | H | 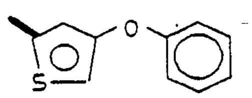 | |

Fortsetzung von Tabelle 2:

| Nr. | $R^1$ | $R^4$ | $R^5$ | Physikalische Daten |
|---|---|---|---|---|
| 252 | " | CN | 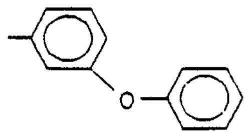 | |
| 253 | 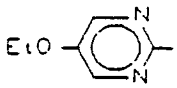 | H | 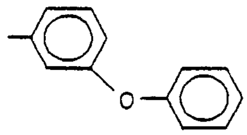 | |
| 254 | " | H | 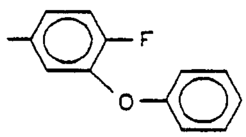 | |
| 255 | " | H | 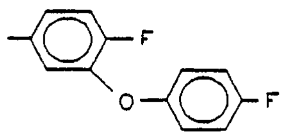 | |
| 256 | " | H | 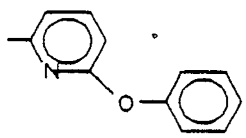 | |
| 257 | " | H | 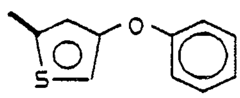 | |
| 258 | " | CN | 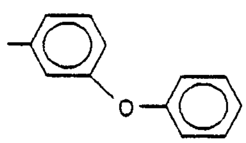 | |

Fortsetzung von Tabelle 2:

| Nr. | $R^1$ | $R^4$ | $R^5$ | Physikalische Daten |
|---|---|---|---|---|
| 259 | 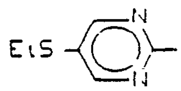 | H | 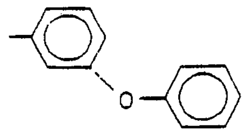 | |
| 260 | " | H | 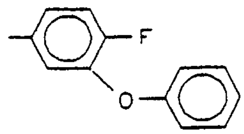 | |
| 261 | " | H | 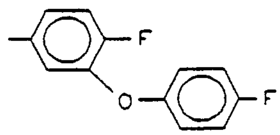 | |
| 262 | " | H | 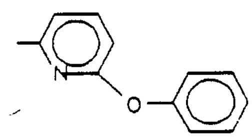 | |
| 263 | " | H | 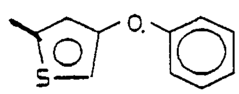 | |
| 264 | " | CN | 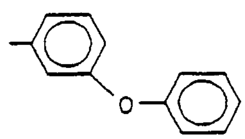 | |
| 265 | 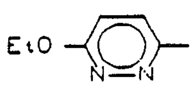 | H | 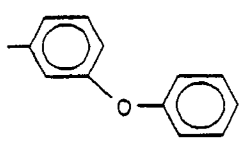 | |

Fortsetzung von Tabelle 2:

| Nr. | $R^1$ | $R^4$ | $R^5$ | Physikalische Daten |
|---|---|---|---|---|
| 266 | " | H | 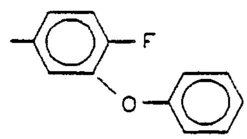 | |
| 267 | " | H | 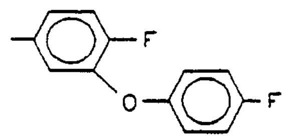 | |
| 268 | " | H | 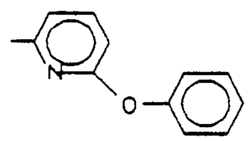 | |
| 269 | " | H | 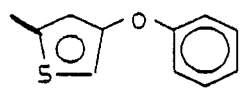 | |
| 270 | " | CN | 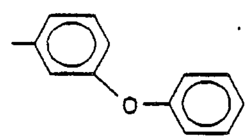 | |
| 271 | 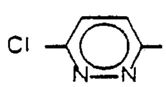 | H | 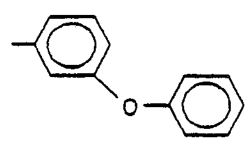 | |
| 272 | " | H | 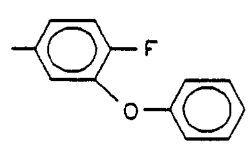 | |

Fortsetzung von Tabelle 2:

| Nr. | $R^1$ | $R^4$ | $R^5$ | Physikalische Daten |
|---|---|---|---|---|
| 273 | " | H | 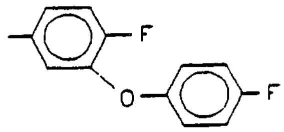 | |
| 274 | " | H | 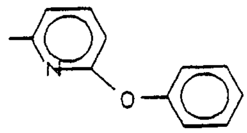 | |
| 275 | " | H | 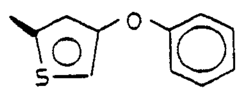 | |
| 276 | " | CN | 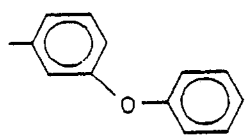 | |
| 277 | 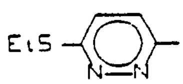 | H | 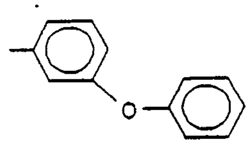 | |
| 278 | " | H | 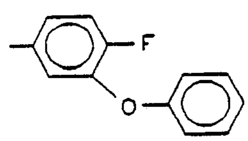 | |
| 279 | " | H | 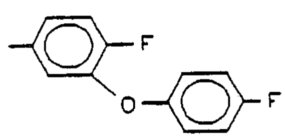 | |

Fortsetzung von Tabelle 2:

| Nr. | $R^1$ | $R^4$ | $R^5$ | Physikalische Daten |
|---|---|---|---|---|
| 280 | " | H | 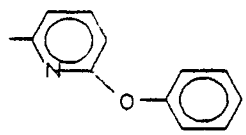 | |
| 281 | " | H | 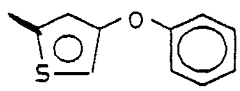 | |
| 282 | " | CN | 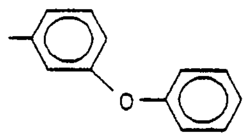 | |
| 283 | 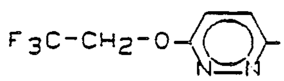 | H | 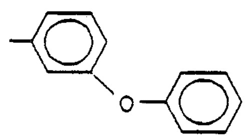 | |
| 284 | " | H | 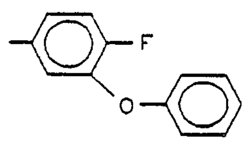 | |
| 285 | " | H | 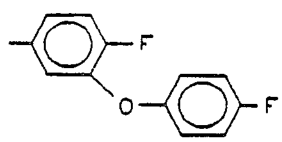 | |
| 286 | " | H | 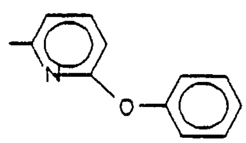 | |

Fortsetzung von Tabelle 2:

| Nr. | $R^1$ | $R^4$ | $R^5$ | Physikalische Daten |
|---|---|---|---|---|
| 287 | " | H | 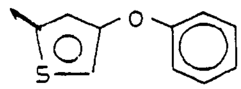 | |
| 288 | " | CN | 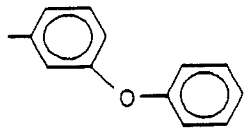 | |
| 289 | 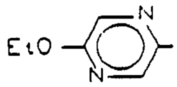 | H | 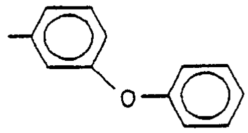 | |
| 290 | " | H | 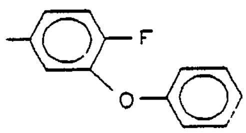 | |
| 291 | " | H | 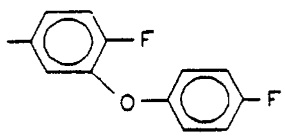 | |
| 292 | " | H | 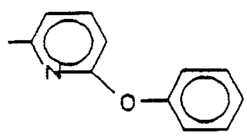 | |
| 293 | " | H | 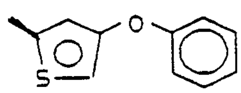 | |

Fortsetzung von Tabelle 2:

| Nr. | $R^1$ | $R^4$ | $R^5$ | Physikalische Daten |
|---|---|---|---|---|
| 294 | " | CN | 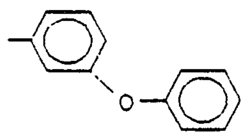 | |
| 295 | 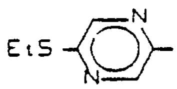 | H | 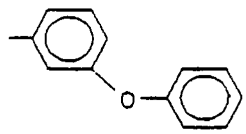 | |
| 296 | " | H | 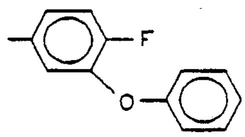 | |
| 297 | " | H | 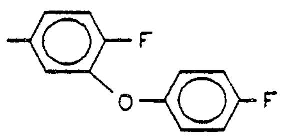 | |
| 298 | " | H | 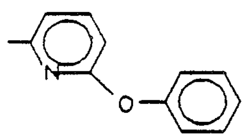 | |
| 299 | " | H | 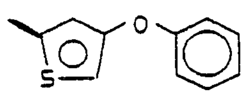 | |
| 300 | " | CN | 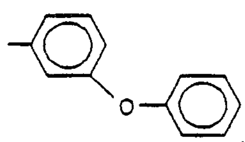 | |

Fortsetzung von Tabelle 2:

| Nr. | $R^1$ | $R^4$ | $R^5$ | Physikalische Daten |
|---|---|---|---|---|
| 301 | 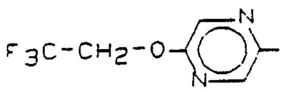 | H | 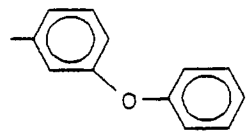 | |
| 302 | " | H | 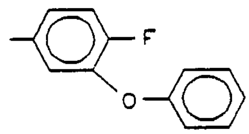 | |
| 303 | " | H | 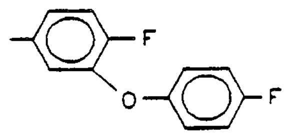 | |
| 304 | " | H | 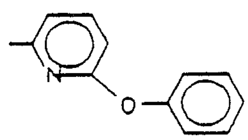 | |
| 305 | " | H | 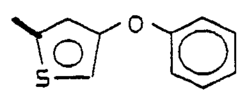 | |
| 306 | " | CN | 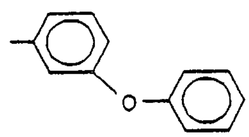 | |
| 307 | 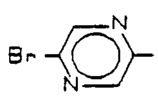 | H | 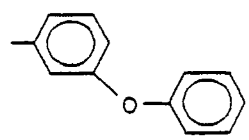 | |

Fortsetzung von Tabelle 2:

| Nr. | $R^1$ | $R^4$ | $R^5$ | Physikalische Daten |
|---|---|---|---|---|
| 308 | " | H | 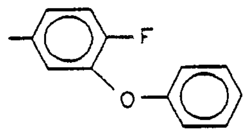 | |
| 309 | " | H | 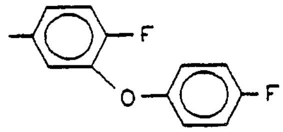 | |
| 310 | " | H | 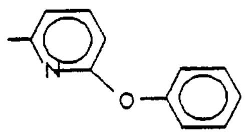 | |
| 311 | " | H | 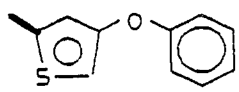 | |
| 312 | " | CN | 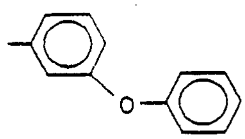 | |
| 313 | 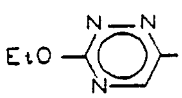 | H | 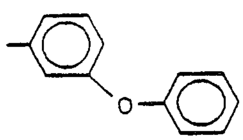 | |
| 314 | " | H | 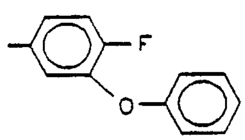 | |

**Fortsetzung von Tabelle 2:**

| Nr. | $R^1$ | $R^4$ | $R^5$ | Physikalische Daten |
|---|---|---|---|---|
| 315 | " | H | 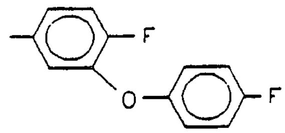 | |
| 316 | " | H | 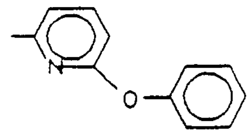 | |
| 317 | " | H | 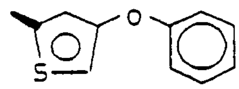 | |
| 318 | " | CN | 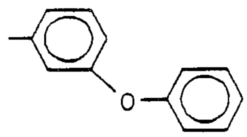 | |
| 319 | 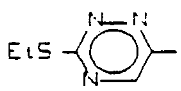 | H | 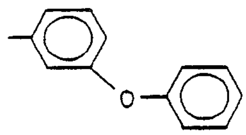 | |
| 320 | " | H | 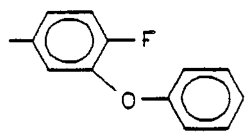 | |
| 321 | " | H | 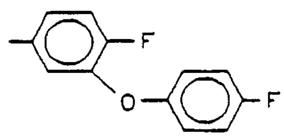 | |

Fortsetzung von Tabelle 2:

| Nr. | $R^1$ | $R^4$ | $R^5$ | Physikalische Daten |
|---|---|---|---|---|
| 322 | " | H | 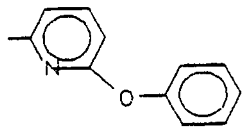 | |
| 323 | " | H | 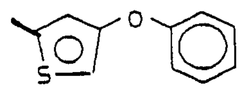 | |
| 324 | " | CN | 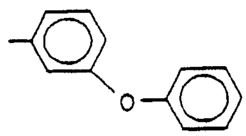 | |
| 325 | $F_3C-CH_2-O-$ 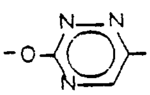 | H | 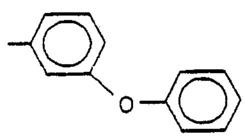 | |
| 326 | " | H | 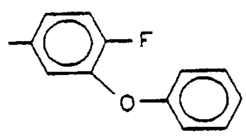 | |
| 327 | " | H | 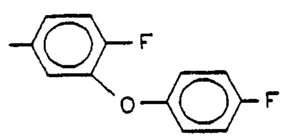 | |
| 328 | " | H | 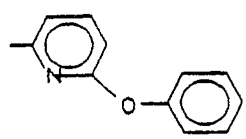 | |

Fortsetzung von Tabelle 2:

| Nr. | $R^1$ | $R^4$ | $R^5$ | Physikalische Daten |
|---|---|---|---|---|
| 329 | " | H | 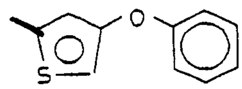 | |
| 330 | " | CN | 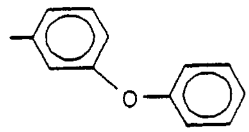 | |
| 331 | 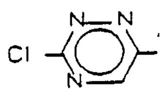 | H | 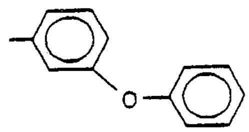 | |
| 332 | " | H | 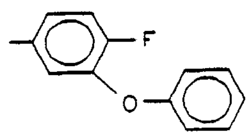 | |
| 333 | " | H | 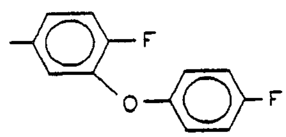 | |
| 334 | " | H | 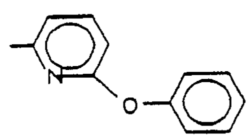 | |
| 335 | " | H | 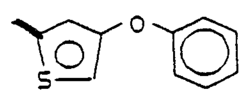 | |

Fortsetzung von Tabelle 2:

| Nr. | $R^1$ | $R^4$ | $R^5$ | Physikalische Daten |
|---|---|---|---|---|
| 336 | " | CN | 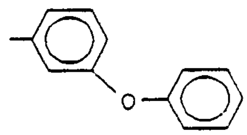 | |
| 337 | 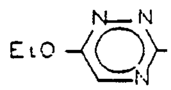 | H | 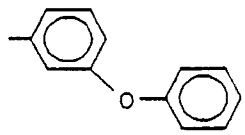 | |
| 338 | " | H | 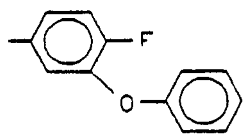 | |
| 339 | " | H | 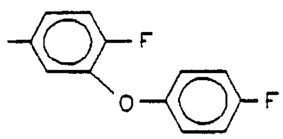 | |
| 340 | " | H | 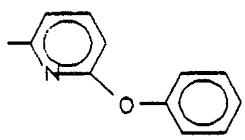 | |
| 341 | " | H | 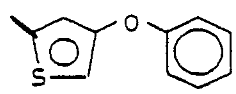 | |
| 342 | " | CN | 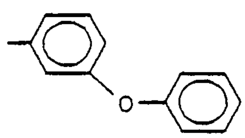 | |

Fortsetzung von Tabelle 2:

| Nr. | $R^1$ | $R^4$ | $R^5$ | Physikalische Daten |
|---|---|---|---|---|
| 343 | 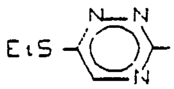 | H | 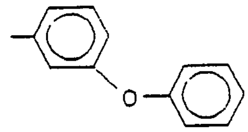 | |
| 344 | " | H | 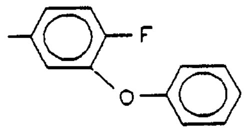 | |
| 345 | " | H | 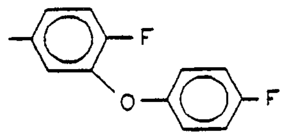 | |
| 346 | " | H | 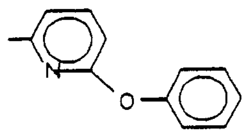 | |
| 347 | " | H | 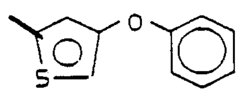 | |
| 348 | " | CN | 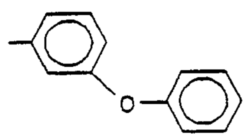 | |
| 349 | 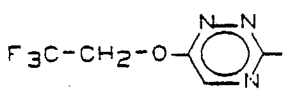 | H | 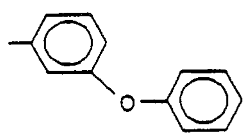 | |

Fortsetzung von Tabelle 2:

| Nr. | $R^1$ | $R^4$ | $R^5$ | Physikalische Daten |
|---|---|---|---|---|
| 350 | " | H | 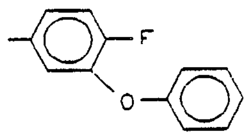 | |
| 351 | " | H | 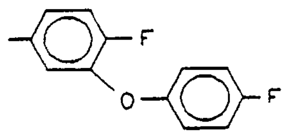 | |
| 352 | " | H | 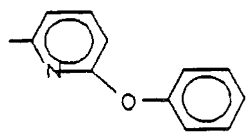 | |
| 353 | " | H | 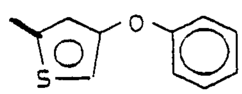 | |
| 354 | " | CN | 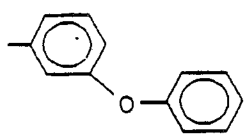 | |
| 355 | 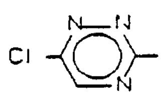 | H | 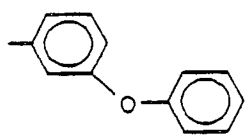 | |
| 356 | " | H | 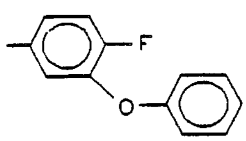 | |

Fortsetzung von Tabelle 2:

| Nr. | $R^1$ | $R^4$ | $R^5$ | Physikalische Daten |
|---|---|---|---|---|
| 357 | " | H | 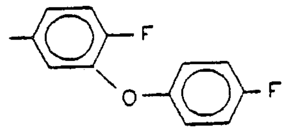 | |
| 358 | " | H | 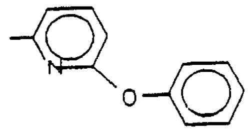 | |
| 359 | " | H | 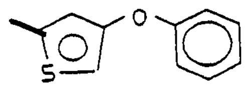 | |
| 360 | " | CN | 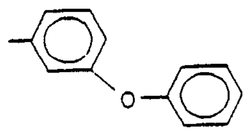 | |
| 361 | 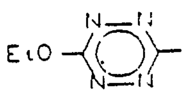 | H | 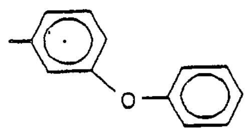 | |
| 362 | " | H | 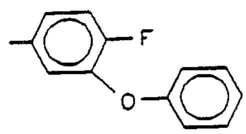 | |
| 363 | " | H | 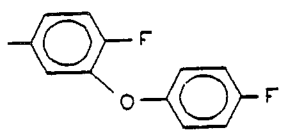 | |

Fortsetzung von Tabelle 2:

| Nr. | $R^1$ | $R^4$ | $R^5$ | Physikalische Daten |
|---|---|---|---|---|
| 364 | " | H | 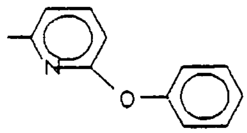 | |
| 365 | " | H | 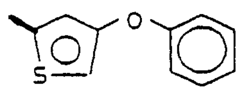 | |
| 366 | " | CN | 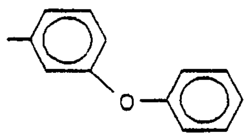 | |
| 367 | 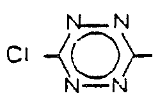 | H | 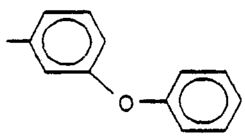 | |
| 368 | " | H | 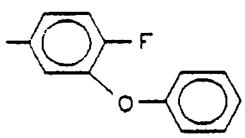 | |
| 369 | " | H | 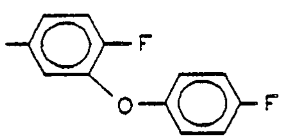 | |
| 370 | " | H | 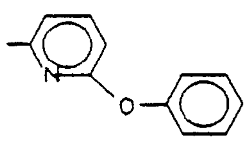 | |

Fortsetzung von Tabelle 2:

| Nr. | $R^1$ | $R^4$ | $R^5$ | Physikalische Daten |
|---|---|---|---|---|
| 371 | " | H | 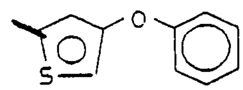 | |
| 372 | " | CN | 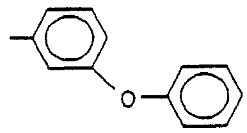 | |
| 373 | 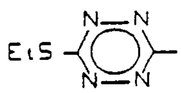 | H | 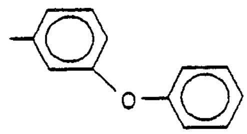 | |
| 374 | " | H | 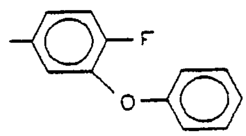 | |
| 375 | " | H | 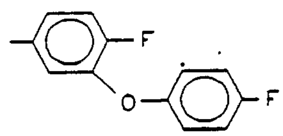 | |
| 376 | " | H | 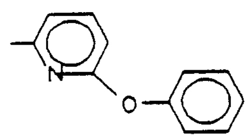 | |
| 377 | " | H | 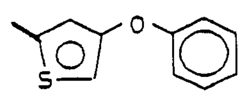 | |

Fortsetzung von Tabelle 2:

| Nr. | $R^1$ | $R^4$ | $R^5$ | Physikalische Daten |
|---|---|---|---|---|
| 378 | " | CN | 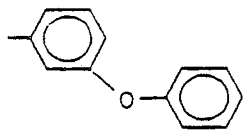 | |
| 379 | 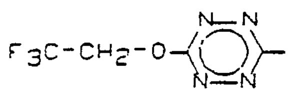 | H | 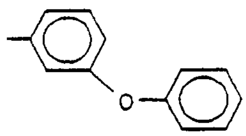 | |
| 380 | " | H | 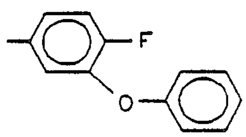 | |
| 381 | " | H | 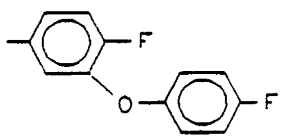 | |
| 382 | " | H | 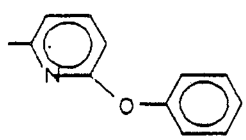 | |
| 383 | " | H | 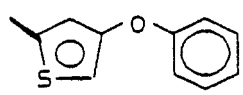 | |
| 384 | " | CN | 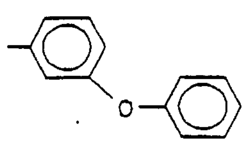 | |

**Tabelle 3:** M = C; $R^2 — R^3$ = $-CH_2-CH_2-$

| Nr. | $R^1$ | $R^4$ | $R^5$ | Physikalische Daten |
|---|---|---|---|---|
| 385 | 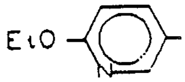 | H | 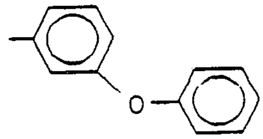 | |
| 386 | " | H | 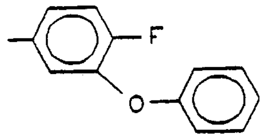 | |
| 387 | " | H | 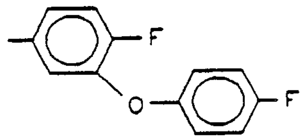 | |
| 388 | " | H | 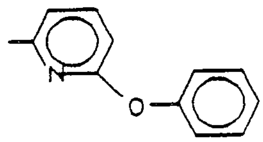 | |
| 389 | " | H | 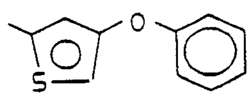 | |
| 390 | " | CN | 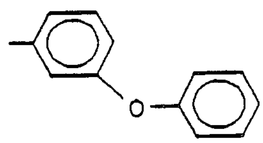 | |
| 391 | 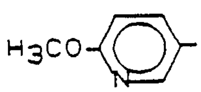 | H | 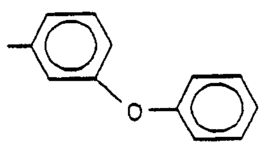 | |

Fortsetzung von Tabelle 3:

| Nr. | $R^1$ | $R^4$ | $R^5$ | Physikalische Daten |
| --- | --- | --- | --- | --- |
| 392 | " | H | 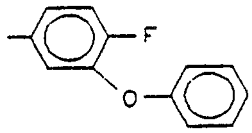 | |
| 393 | " | H | 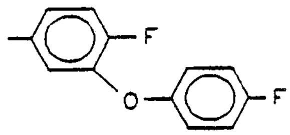 | |
| 394 | " | H | 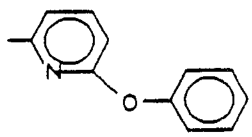 | |
| 395 | " | H | 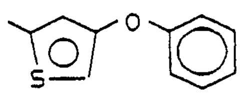 | |
| 396 | " | CN | 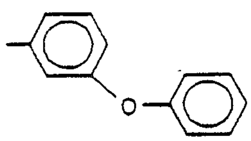 | |
| 397 | 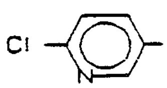 | H | 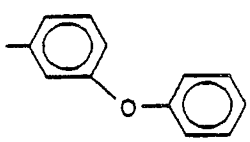 | |
| 398 | " | H | 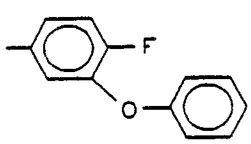 | |

Fortsetzung von Tabelle 3:

| Nr. | $R^1$ | $R^4$ | $R^5$ | Physikalische Daten |
|---|---|---|---|---|
| 399 | " | H | 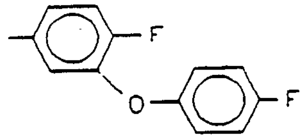 | |
| 400 | " | H | 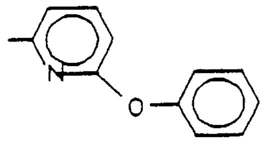 | |
| 401 | " | H | 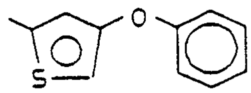 | |
| 402 | " | CN | 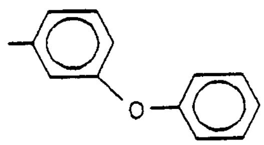 | |
| 403 | 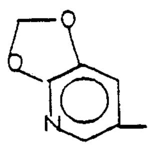 | H | 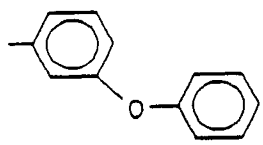 | |
| 404 | " | H | 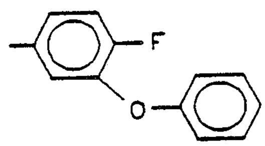 | |
| 405 | " | H | 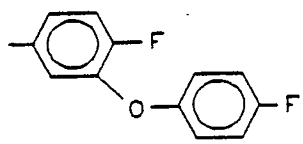 | |

Fortsetzung von Tabelle 3:

| Nr. | $R^1$ | $R^4$ | $R^5$ | Physikalische Daten |
|---|---|---|---|---|
| 406 | " | H | 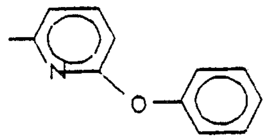 | |
| 407 | " | H | 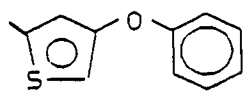 | |
| 408 | " | CN | 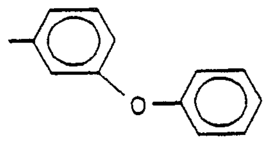 | |
| 409 | 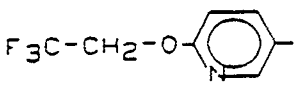 | H | 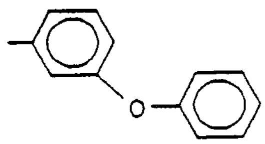 | |
| 410 | " | H | 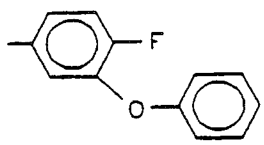 | |
| 411 | " | H | 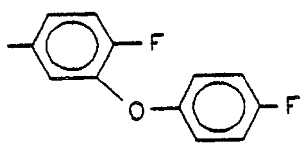 | |
| 412 | " | H | 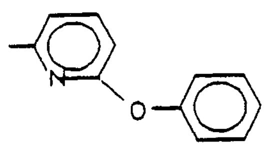 | |

**Fortsetzung von Tabelle 3:**

| Nr. | $R^1$ | $R^4$ | $R^5$ | Physikalische Daten |
| --- | --- | --- | --- | --- |
| 413 | " | H | 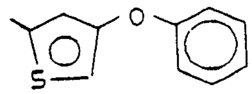 | |
| 414 | " | CN | 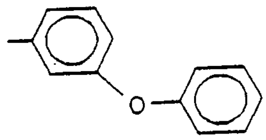 | |
| 415 | 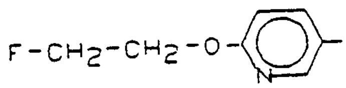 | H | 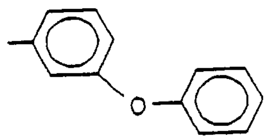 | |
| 416 | " | H | 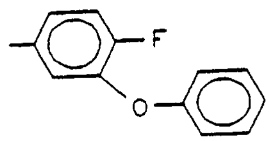 | |
| 417 | " | H | 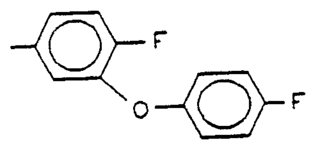 | |
| 418 | " | H | 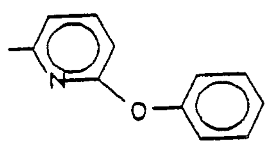 | |
| 419 | " | H | 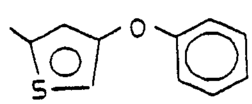 | |

Fortsetzung von Tabelle 3:

| Nr. | $R^1$ | $R^4$ | $R^5$ | Physikalische Daten |
|---|---|---|---|---|
| 420 | " | CN | 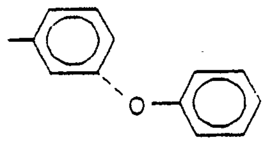 | |
| 421 | 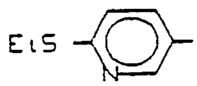 | H | 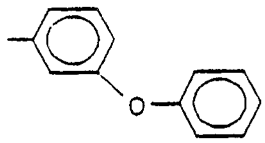 | |
| 422 | " | H | 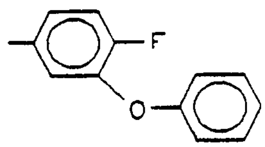 | |
| 423 | " | H | 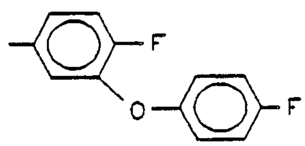 | |
| 424 | " | H | 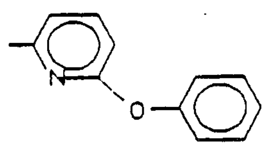 | |
| 425 | " | H | 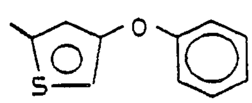 | |
| 426 | " | CN | 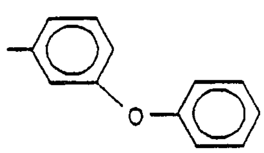 | |

Fortsetzung von Tabelle 3:

| Nr. | $R^1$ | $R^4$ | $R^5$ | Physikalische Daten |
|---|---|---|---|---|
| 427 | 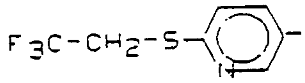 | H | 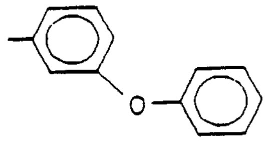 | |
| 428 | " | H | 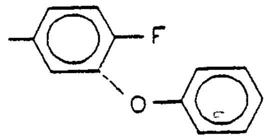 | |
| 429 | " | H | 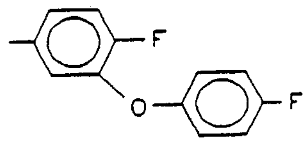 | |
| 430 | " | H | 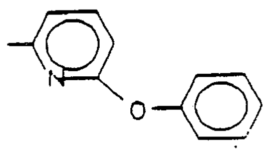 | |
| 431 | " | H | 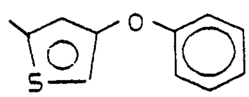 | |
| 432 | " | CN | 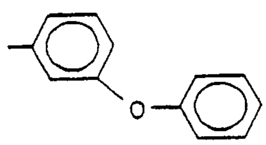 | |
| 433 | 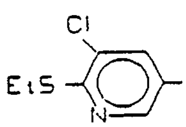 | H | 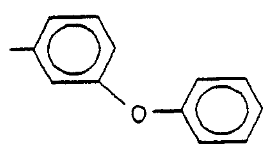 | |

Fortsetzung von Tabelle 3:

| Nr. | $R^1$ | $R^4$ | $R^5$ | Physikalische Daten |
|---|---|---|---|---|
| 434 | " | H | 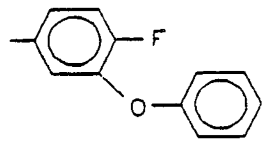 | |
| 435 | " | H | 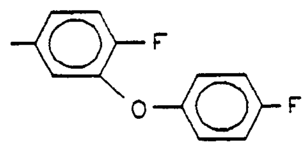 | |
| 436 | " | H | 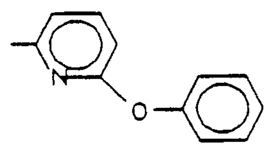 | |
| 437 | " | H | 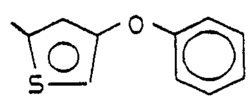 | |
| 438 | " | CN | 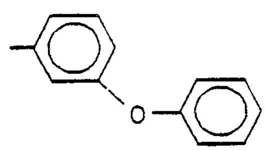 | |
| 439 | 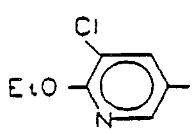 | H | 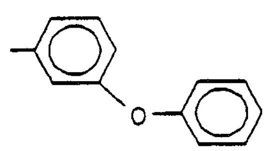 | |
| 440 | " | H | 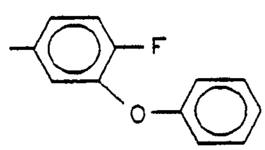 | |

Fortsetzung von Tabelle 3:

| Nr. | $R^1$ | $R^4$ | $R^5$ | Physikalische Daten |
|---|---|---|---|---|
| 441 | " | H | 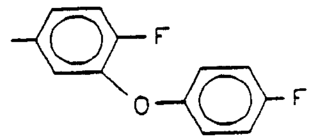 | |
| 442 | " | H | 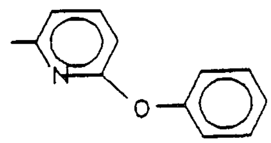 | |
| 443 | " | H | 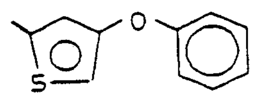 | |
| 444 | " | CN | 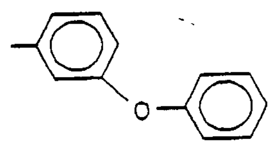 | |
| 445 | 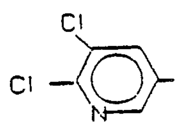 | H | 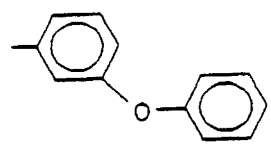 | |
| 446 | " | H | 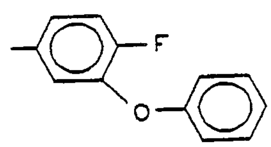 | |
| 447 | " | H | 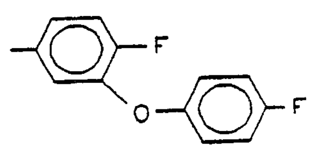 | |

Fortsetzung von Tabelle 3:

| Nr. | $R^1$ | $R^4$ | $R^5$ | Physikalische Daten |
|---|---|---|---|---|
| 448 | " | H | 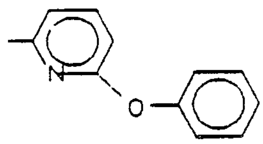 | |
| 449 | " | H | 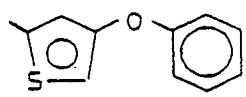 | |
| 450 | " | CN | 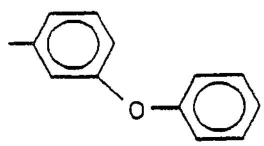 | |
| 451 | 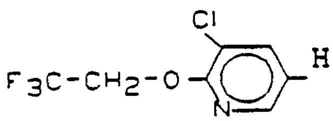 | H | 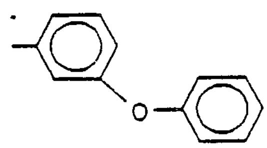 | |
| 452 | " | H | 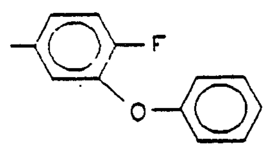 | |
| 453 | " | H | 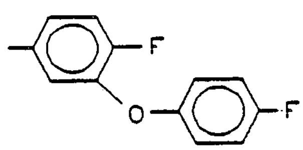 | |
| 454 | " | H | 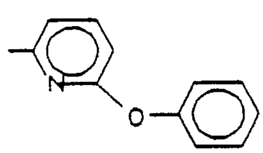 | |

Fortsetzung von Tabelle 3:

| Nr. | $R^1$ | $R^4$ | $R^5$ | Physikalische Daten |
|---|---|---|---|---|
| 455 | " | H | 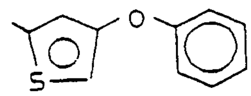 | |
| 456 | " | CN | 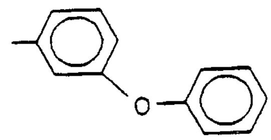 | |
| 457 | 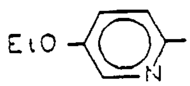 | | 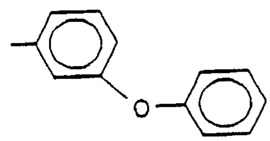 | |
| 458 | " | H | 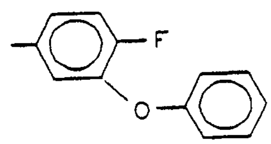 | |
| 459 | " | H | 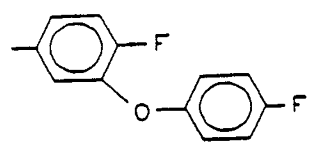 | |
| 460 | " | H | 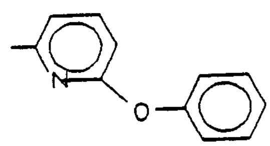 | |
| 461 | " | H | 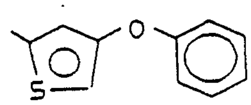 | |

Fortsetzung von Tabelle 3:

| Nr. | $R^1$ | $R^4$ | $R^5$ | Physikalische Daten |
|---|---|---|---|---|
| 462 | " | CN | 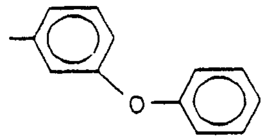 | |
| 463 | 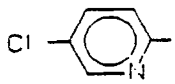 | H | 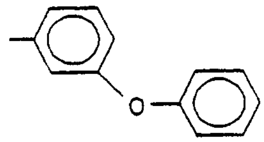 | |
| 464 | " | H | 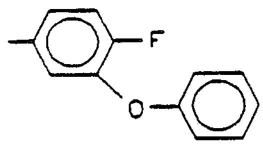 | |
| 465 | " | H | 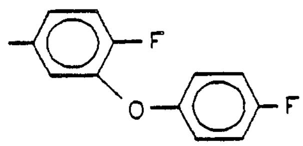 | |
| 466 | " | H | 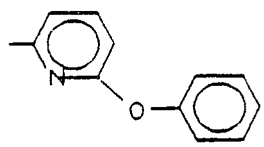 | |
| 467 | " | H | 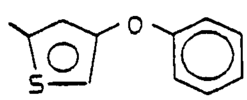 | |
| 468 | " | CN | 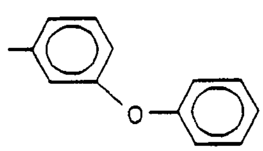 | |

Fortsetzung von Tabelle 3:

| Nr. | $R^1$ | $R^4$ | $R^5$ | Physikalische Daten |
|---|---|---|---|---|
| 469 | 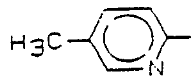 | H | 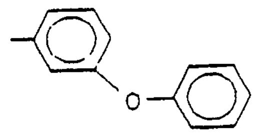 | |
| 470 | " | H | 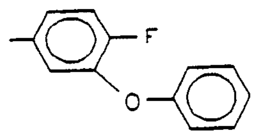 | |
| 471 | " | H | 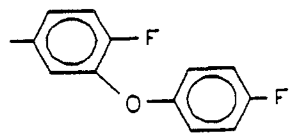 | |
| 472 | " | H | 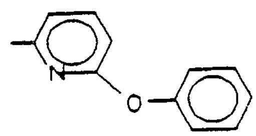 | |
| 473 | " | H | 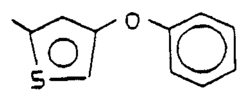 | |
| 474 | " | CN | 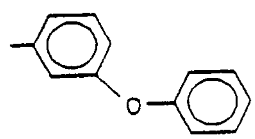 | |
| 475 | 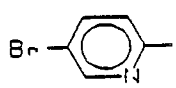 | H | 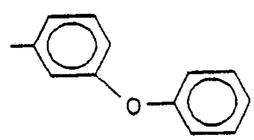 | |

Fortsetzung von Tabelle 3:

| Nr. | $R^1$ | $R^4$ | $R^5$ | Physikalische Daten |
|---|---|---|---|---|
| 476 | " | H | 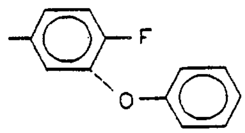 | |
| 477 | " | H | 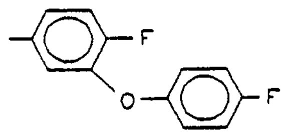 | |
| 478 | " | H | 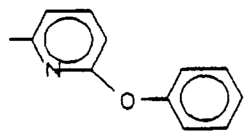 | |
| 479 | " | H | 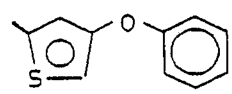 | |
| 480 | " | CN | 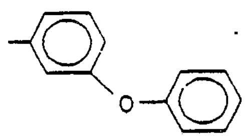 | |
| 481 | 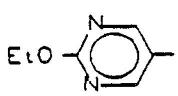 | | 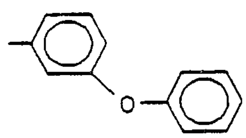 | |
| 482 | " | H | 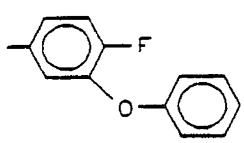 | |

Fortsetzung von Tabelle 3:

| Nr. | $R^1$ | $R^4$ | $R^5$ | Physikalische Daten |
|---|---|---|---|---|
| 483 | " | H | 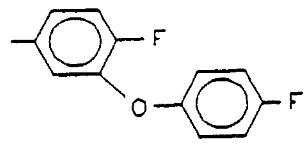 | |
| 484 | " | H | 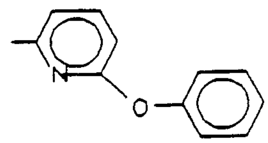 | |
| 485 | " | H | 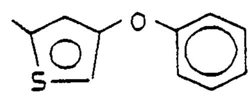 | |
| 486 | " | CN | 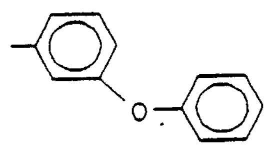 | |
| 487 | 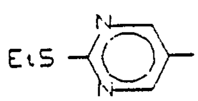 | H | 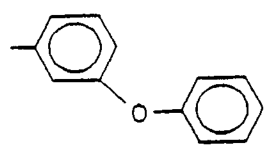 | |
| 488 | " | H | 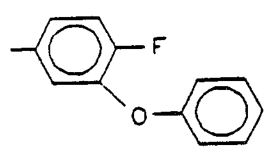 | |
| 489 | " | H | 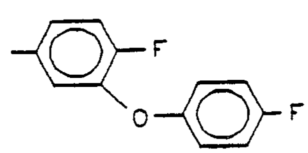 | |

Fortsetzung von Tabelle 3:

| Nr. | $R^1$ | $R^4$ | $R^5$ | Physikalische Daten |
|---|---|---|---|---|
| 490 | " | H | 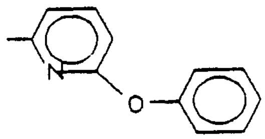 | |
| 491 | " | H | 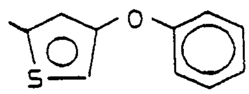 | |
| 492 | " | CN | 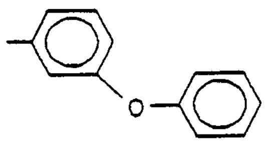 | |
| 493 | $F_3C-CH_2-O-$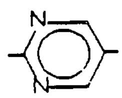 | | 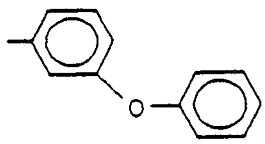 | |
| 494 | " | H | 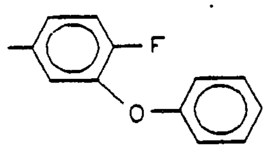 | |
| 495 | " | H | 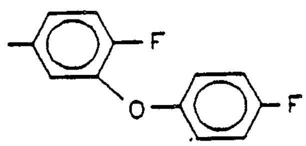 | |
| 496 | " | H | 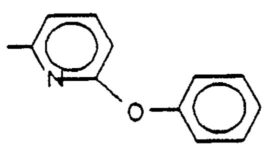 | |

Fortsetzung von Tabelle 3:

| Nr. | $R^1$ | $R^4$ | $R^5$ | Physikalische Daten |
|---|---|---|---|---|
| 497 | " | H | 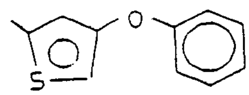 | |
| 498 | " | CN | 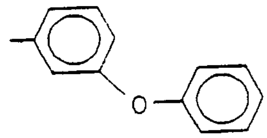 | |
| 499 | $F-CH_2-CH_2-O-$ 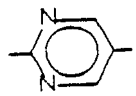 | H | 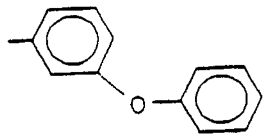 | |
| 500 | " | H | 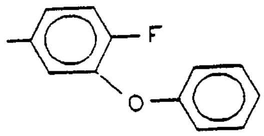 | |
| 501 | " | H | 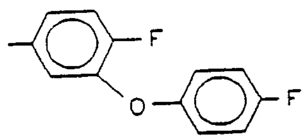 | |
| 502 | " | H | 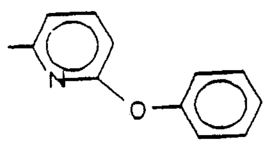 | |
| 503 | " | H | 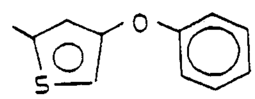 | |

**Fortsetzung von Tabelle 3:**

| Nr. | $R^1$ | $R^4$ | $R^5$ | Physikalische Daten |
|---|---|---|---|---|
| 504 | " | CN | 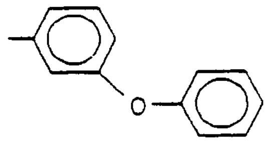 | |
| 505 | 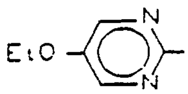 | H | 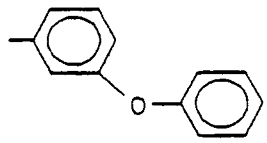 | |
| 506 | " | H | 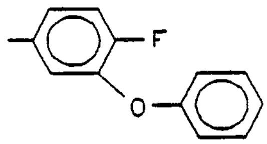 | |
| 507 | " | H | 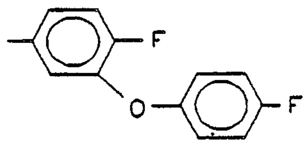 | |
| 508 | " | H | 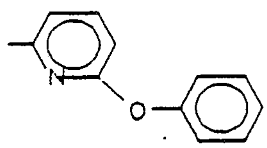 | |
| 509 | " | H | 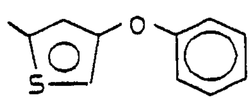 | |
| 510 | " | CN | 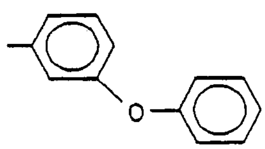 | |

Fortsetzung von Tabelle 3:

| Nr. | $R^1$ | $R^4$ | $R^5$ | Physikalische Daten |
|---|---|---|---|---|
| 511 | 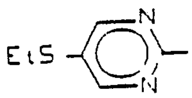 | H | 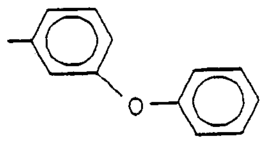 | |
| 512 | " | H | 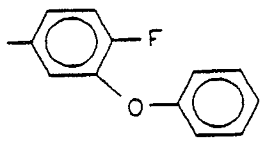 | |
| 513 | " | H | 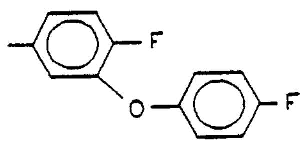 | |
| 514 | " | H | 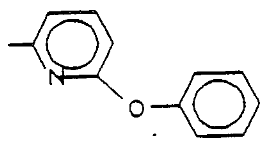 | |
| 515 | " | H | 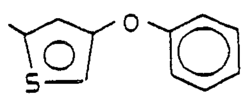 | |
| 516 | " | CN | 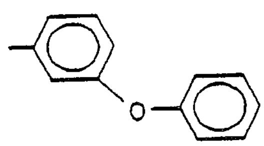 | |
| 517 | 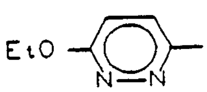 | H | 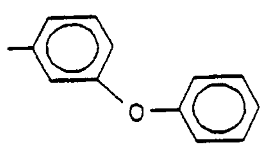 | |

Fortsetzung von Tabelle 3:

| Nr. | $R^1$ | $R^4$ | $R^5$ | Physikalische Daten |
|---|---|---|---|---|
| 518 | " | H | 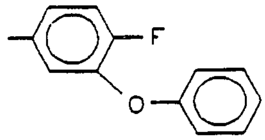 | |
| 519 | " | H | 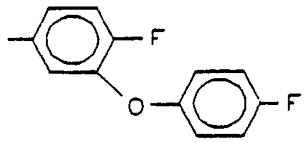 | |
| 520 | " | H | 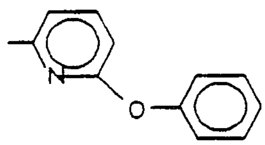 | |
| 521 | " | H | 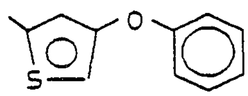 | |
| 522 | " | CN | 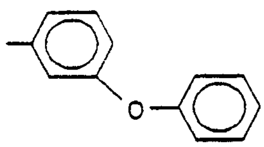 | |
| 523 | 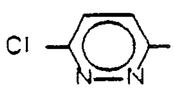 | H | 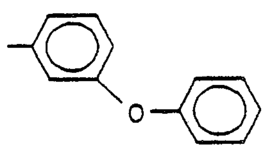 | |
| 524 | " | H | 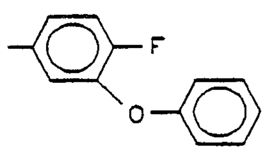 | |

Fortsetzung von Tabelle 3:

| Nr. | $R^1$ | $R^4$ | $R^5$ | Physikalische Daten |
|---|---|---|---|---|
| 525 | " | H | 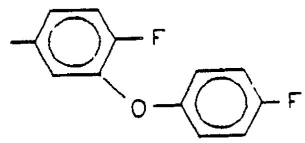 | |
| 526 | " | H | 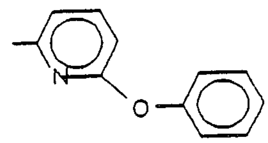 | |
| 527 | " | H | 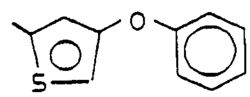 | |
| 528 | " | CN | 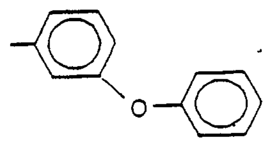 | |
| 529 | 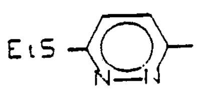 | H | 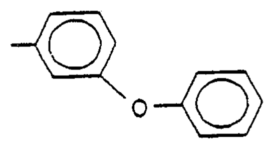 | |
| 530 | " | H | 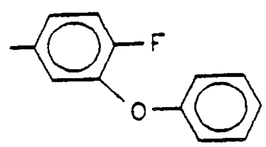 | |
| 531 | " | H | 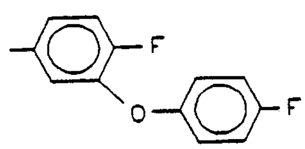 | |

Fortsetzung von Tabelle 3:

| Nr. | $R^1$ | $R^4$ | $R^5$ | Physikalische Daten |
|---|---|---|---|---|
| 532 | " | H | 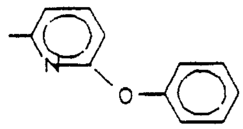 | |
| 533 | " | H | 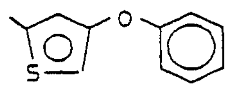 | |
| 534 | " | CN | 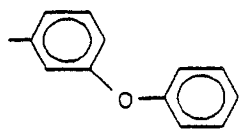 | |
| 535 | 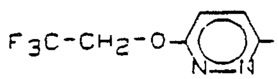 | H | 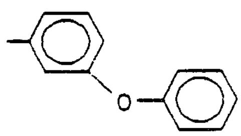 | |
| 536 | " | H | 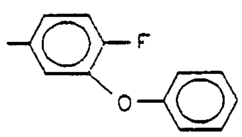 | |
| 537 | " | H | 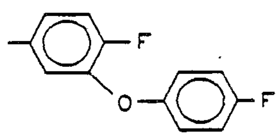 | |
| 538 | " | H | 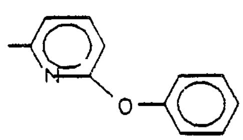 | |

Fortsetzung von Tabelle 3:

| Nr. | $R^1$ | $R^4$ | $R^5$ | Physikalische Daten |
|---|---|---|---|---|
| 539 | " | H | 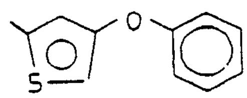 | |
| 540 | " | CN | 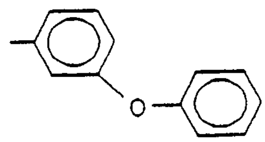 | |
| 541 | 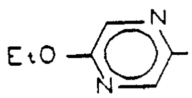 | H | 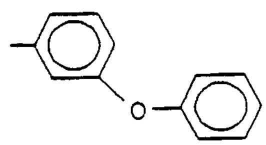 | |
| 542 | " | H | 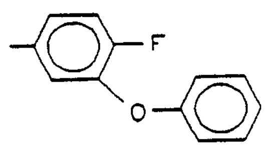 | |
| 543 | " | H | 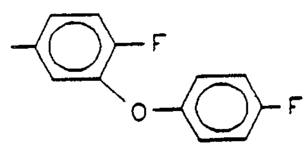 | |
| 544 | " | H | 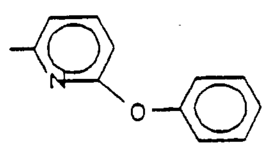 | |
| 545 | " | H | 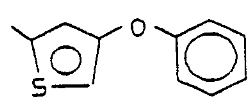 | |

Fortsetzung von Tabelle 3:

| Nr. | $R^1$ | $R^4$ | $R^5$ | Physikalische Daten |
|---|---|---|---|---|
| 546 | " | CN | 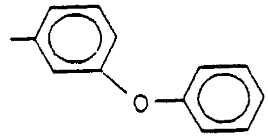 | |
| 547 | 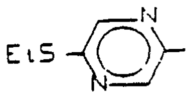 | H | 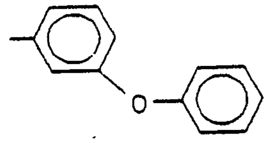 | |
| 548 | " | H | 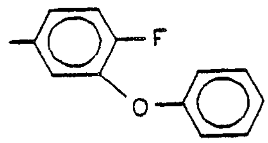 | |
| 549 | " | H | 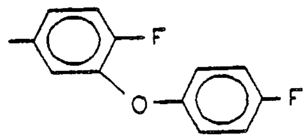 | |
| 550 | " | H | 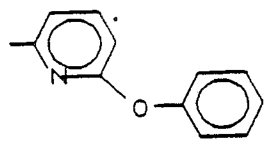 | |
| 551 | " | H | 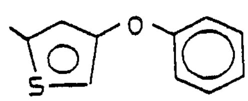 | |
| 552 | " | CN | 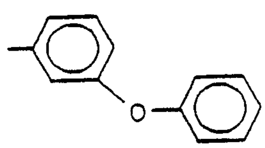 | |

Fortsetzung von Tabelle 3:

| Nr. | $R^1$ | $R^4$ | $R^5$ | Physikalische Daten |
|---|---|---|---|---|
| 553 | 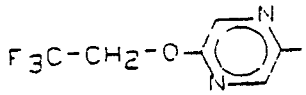 | H | 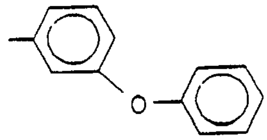 | |
| 554 | " | H | 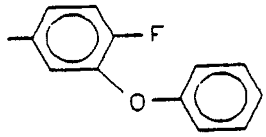 | |
| 555 | " | H | 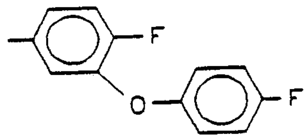 | |
| 556 | " | H | 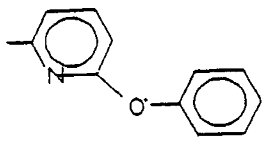 | |
| 557 | " | H | 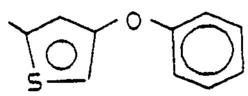 | |
| 558 | " | CN | 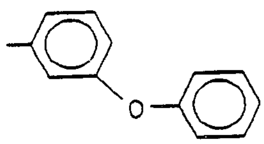 | |
| 559 | 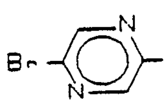 | H | 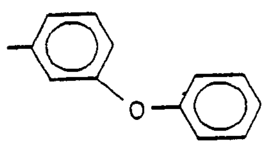 | |

Fortsetzung von Tabelle 3:

| Nr. | $R^1$ | $R^4$ | $R^5$ | Physikalische Daten |
|---|---|---|---|---|
| 560 | " | H | 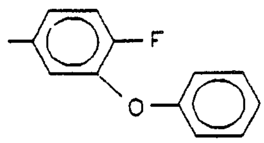 | |
| 561 | " | H | 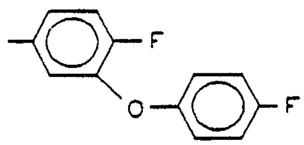 | |
| 562 | " | H | 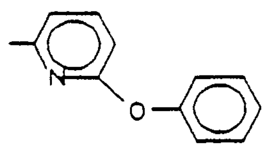 | |
| 563 | " | H | 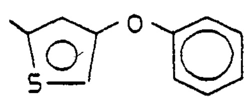 | |
| 564 | " | CN | 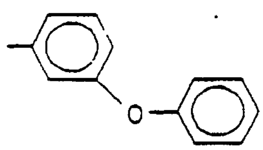 | |
| 565 | 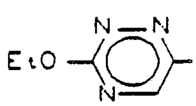 | H | 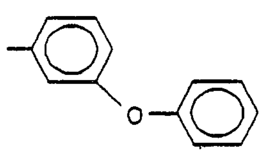 | |
| 566 | " | H | 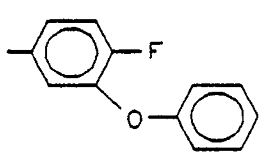 | |

Fortsetzung von Tabelle 3:

| Nr. | $R^1$ | $R^4$ | $R^5$ | Physikalische Daten |
| --- | --- | --- | --- | --- |
| 567 | " | H | 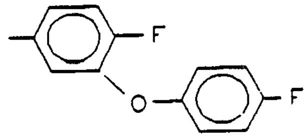 | |
| 568 | " | H | 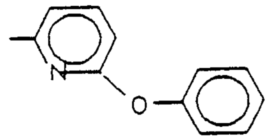 | |
| 569 | " | H | 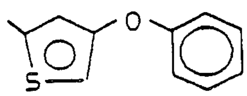 | |
| 570 | " | CN | 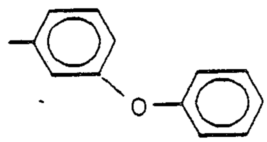 | |
| 571 | 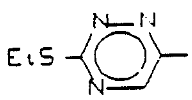 | H | 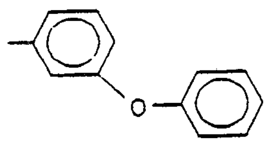 | |
| 572 | " | H | 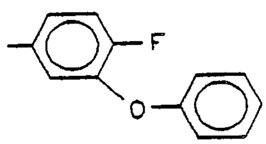 | |
| 573 | " | H | 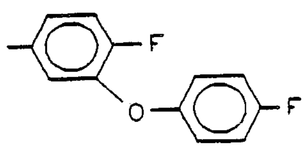 | |

Fortsetzung von Tabelle 3:

| Nr. | $R^1$ | $R^4$ | $R^5$ | Physikalische Daten |
|---|---|---|---|---|
| 574 | " | H | 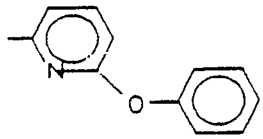 | |
| 575 | " | H | 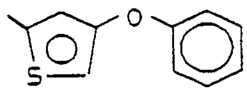 | |
| 576 | " | CN | 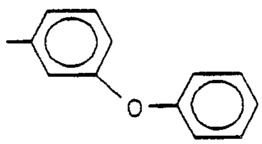 | |
| 577 | $F_3C-CH_2-O-$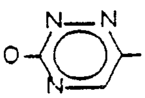 | H | 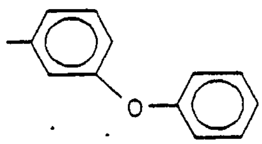 | |
| 578 | " | H | 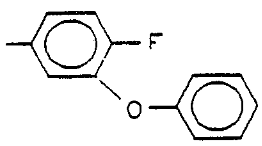 | |
| 579 | " | H | 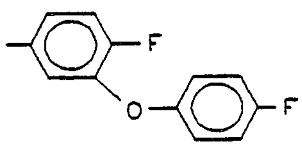 | |
| 580 | " | H | 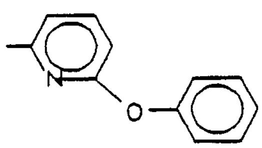 | |

Fortsetzung von Tabelle 3:

| Nr. | $R^1$ | $R^4$ | $R^5$ | Physikalische Daten |
|---|---|---|---|---|
| 581 | " | H | 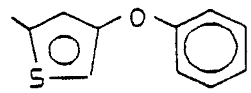 | |
| 582 | " | CN | 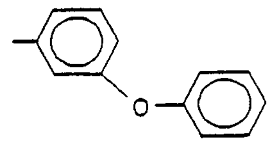 | |
| 583 | 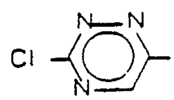 | H | 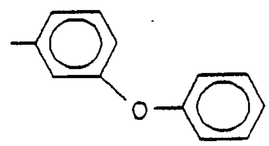 | |
| 584 | " | H | 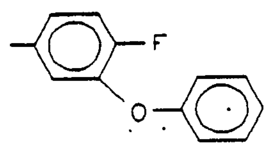 | |
| 585 | " | H | 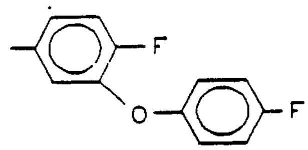 | |
| 586 | " | H | 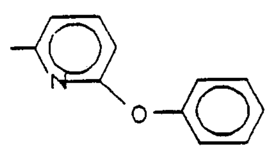 | |
| 587 | " | H | 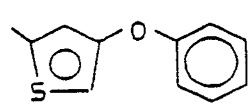 | |

Fortsetzung von Tabelle 3:

| Nr. | $R^1$ | $R^4$ | $R^5$ | Physikalische Daten |
|---|---|---|---|---|
| 588 | " | CN | 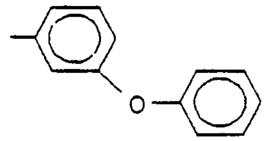 | |
| 589 | 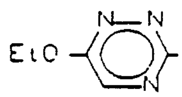 | H | 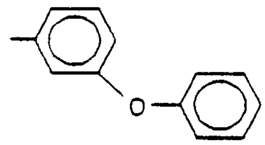 | |
| 590 | " | H | 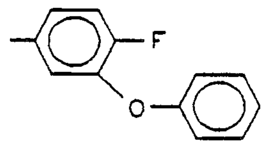 | |
| 591 | " | H | 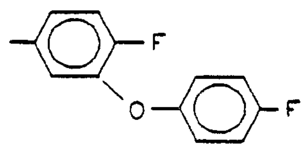 | |
| 592 | " | H | 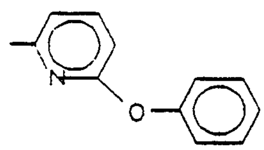 | |
| 593 | " | H | 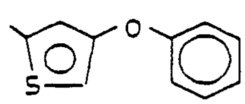 | |
| 594 | " | CN | 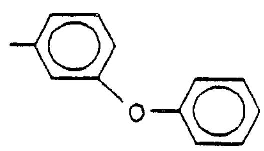 | |

Fortsetzung von Tabelle 3:

| Nr. | $R^1$ | $R^4$ | $R^5$ | Physikalische Daten |
|---|---|---|---|---|
| 595 | 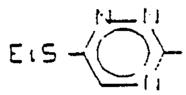 | H | 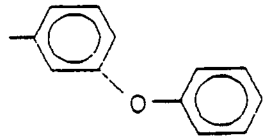 | |
| 596 | " | H | 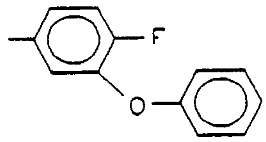 | |
| 597 | " | H | 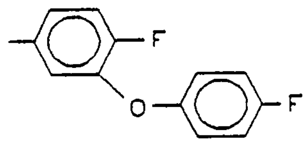 | |
| 598 | " | H | 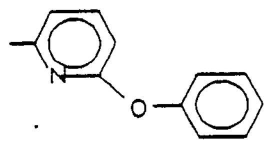 | |
| 599 | " | H | 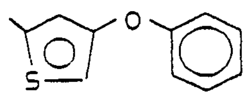 | |
| 600 | " | CN | 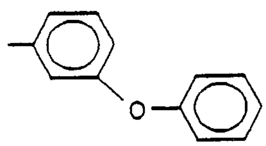 | |
| 601 | 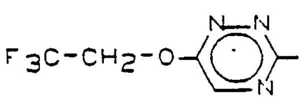 | H | 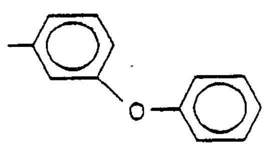 | |

Fortsetzung von Tabelle 3:

| Nr. | $R^1$ | $R^4$ | $R^5$ | Physikalische Daten |
|---|---|---|---|---|
| 602 | " | H | 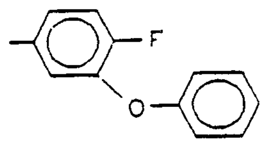 | |
| 603 | " | H | 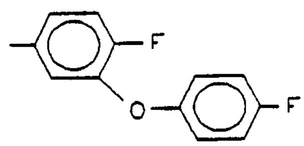 | |
| 604 | " | H | 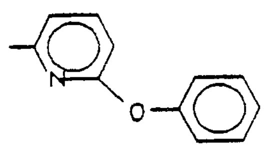 | |
| 605 | " | H | 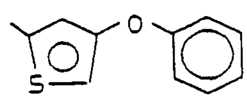 | |
| 606 | " | CN | 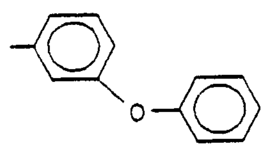 | |
| 607 | 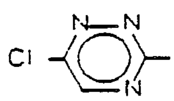 | H | 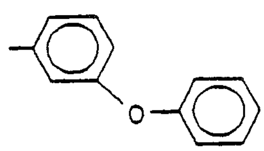 | |
| 608 | " | H | 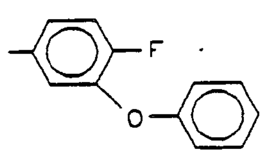 | |

**Fortsetzung von Tabelle 3:**

| Nr. | $R^1$ | $R^4$ | $R^5$ | Physikalische Daten |
|---|---|---|---|---|
| 609 | " | H | 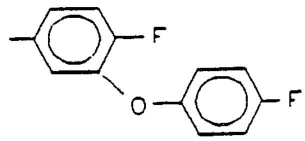 | |
| 610 | " | H | 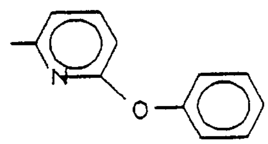 | |
| 611 | " | H | 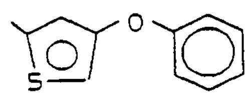 | |
| 612 | " | CN | 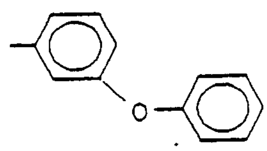 | |
| 613 | 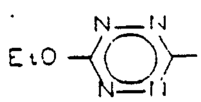 | H | 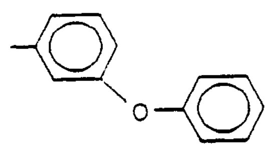 | |
| 614 | " | H | 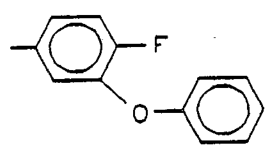 | |
| 615 | " | H | 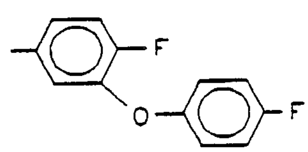 | |

Fortsetzung von Tabelle 3:

| Nr. | $R^1$ | $R^4$ | $R^5$ | Physikalische Daten |
|---|---|---|---|---|
| 616 | " | H | 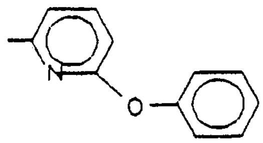 | |
| 617 | " | H | 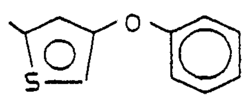 | |
| 618 | " | CN | 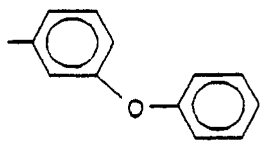 | |
| 619 | 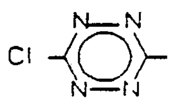 | H | 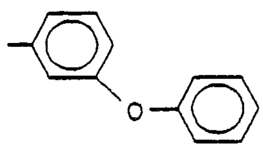 | |
| 620 | " | H | 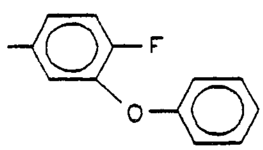 | |
| 621 | " | H | 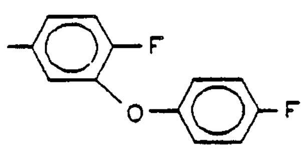 | |
| 622 | " | H | 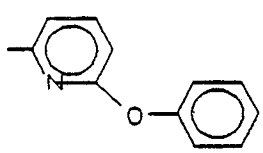 | |

Fortsetzung von Tabelle 3:

| Nr. | $R^1$ | $R^4$ | $R^5$ | Physikalische Daten |
|---|---|---|---|---|
| 623 | " | H | 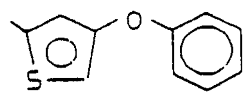 | |
| 624 | " | CN | 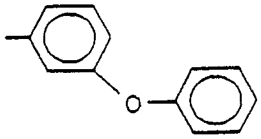 | |
| 625 | 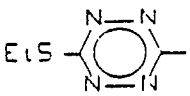 | H | 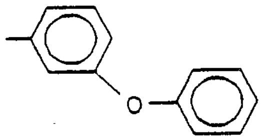 | |
| 626 | " | H | 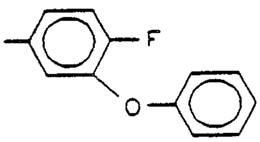 | |
| 627 | " | H | 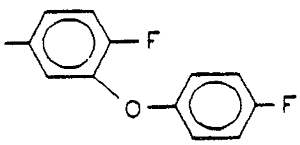 | |
| 628 | " | H | 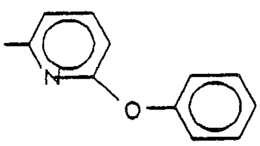 | |
| 629 | " | H | 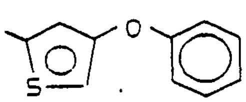 | |

Fortsetzung von Tabelle 3:

| Nr. | $R^1$ | $R^4$ | $R^5$ | Physikalische Daten |
|---|---|---|---|---|
| 630 | " | CN | 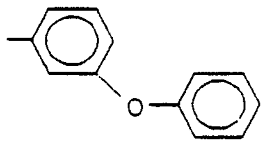 | |
| 631 | 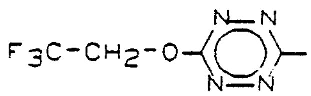 | H | 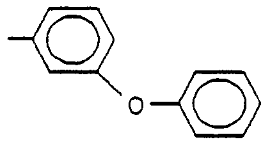 | |
| 632 | " | H | 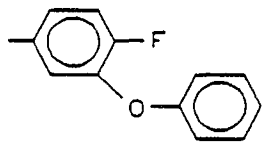 | |
| 633 | " | H | 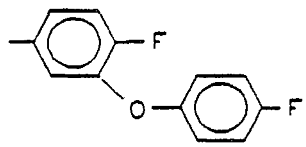 | |
| 634 | " | H | 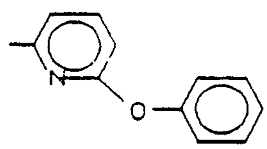 | |
| 635 | " | H | 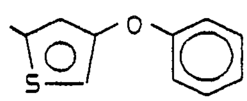 | |
| 636 | " | CN | 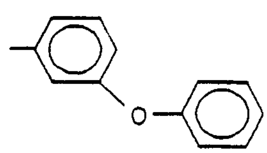 | |

## Biologische Beispiele

### Beispiel 1

Mit Bohnenspinnmilben (Tetranychus urticae, Vollpopulation) stark befallene Bohnenpflanzen (Phaseolus v.) wurden mit der wäßrigen Verdünnung eines Emulsionskonzentrates, das 1000 ppm des jeweiligen

Wirkstoffes enthielt, gespritzt.

Die Mortalität der Milben wurde nach 7 Tagen kontrolliert. 100 %ige Abtötung wurde mit den Verbindungen gemäß Beispiel 25, 26, 157 und 158 erzielt.

**Beispiel 2**

Mit Kundebohnenblattlaus (Aphis craccivora) stark besetzte Ackerbohnen (Vicia faba) wurden mit wäßrigen Verdünnungen von Spritzpulverkonzentraten mit 100 ppm Wirkstoffgehalt bis zum Stadium des beginnenden Abtropfens besprüht.

Die Mortalität der Blattläuse wurde nach 3 Tagen bestimmt. Eine 100 %ige Abtötung konnte mit den Verbindungen gemäß Beispiel 1, 25, 26, 133, 157 und 158 erzielt werden.

**Beispiel 3**

Die Bodeninnenseiten von mit künstlichem Nährmedium beschichteten Petrischalen wurden nach dem Erstarren des Futterbreis mit jeweils 3 ml einer 1000 ppm an Wirkstoff enthaltenden Emulsion besprüht. Nach Abtrocknen des Spritzbelages und Einsetzen von 10 Larven des gemeinen Baumwollwurmes (Prodenia litura) wurden die Schalen 7 Tage bei 21 °C aufbewahrt und der Wirkungsgrad der jeweiligen Verbindungen (ausgedrückt in % Mortalität) bestimmt. Die Verbindungen 1, 2, 25, 26, 133, 134, 157 und 158 ergaben in diesem Test eine Wirksamkeit von jeweils 100 %.

**Beispiel 4**

Blätter der Bohne (Phaseolus vulgaris) wurden mit einer wäßrigen Emulsion der Verbindungen in einer Wirkstoffkonzentration von 250 ppm gleichmäßig besprüht und zu gleichbehandelten Larven des mexikanischen Bohnenkäfers (Epilachna varivestis) in Beobachtungskäfige gestellt. Die Verbindungen 1, 2, 25, 26, 133, 134, 157 und 158 zeigten nach 48 h eine 100 %ige Abtötung der Testinsekten.

**Beispiel 5**

Die Boden- und Deckelinnenseiten von Glaspetrischalen wurden mit Filterpapierscheiben ausgekleidet und diese mit je 2 ml einer 500 ppm Wirkstoff enthaltenden wäßrigen Emulsion der zu prüfenden Verbindungen besprüht. Anschließend wurden je 20 geflügelte Exemplare der braunrückigen Reiszikade (Nilaparvata lugens) eingesetzt, die Böden mit den Deckeln verschlossen und die Petrischalen bei 22 °C 48 h aufbewahrt.

Nach dieser Zeigt ergaben die geprüften Verbindungen 1, 2, 25, 26, 133, 134, 157 und 158 bei den Versuchstieren jeweils 100 %ige Mortalität.

**Ansprüche**

1. Verbindungen der Formel I, ihre optischen Isomeren und deren Gemische,

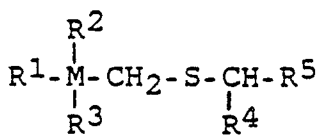

(I),

worin

M = C oder Si,

$R^1$ = unsubstituiertes oder substituiertes Pyridyl, unsubstituiertes oder substituiertes Pyrimidyl, sowie - für M = C - unsubstituiertes oder substituiertes Pyridazinyl, unsubstituiertes oder substituiertes Pyrazinyl, unsubstituiertes oder substituiertes 1,2,4-Triazinyl, unsubstituiertes oder substituiertes 1,2,4,5-Tetrazinyl,

$R^2$, $R^3$ = unabhängig voneinander ($C_1$-$C_3$-)Alkyl, ($C_2$-$C_8$) Alkenyl, ($C_1$-$C_2$)Haloalkyl, Phenyl oder $R^2$ und $R^3$ eine Alkylenkette, die - zusammen mit dem quartären Zentralatom (M) einen unsubstituierten oder fluorsubstituierten Ring mit vier bis sechs Ringgliedern (für M = Si) oder mit drei bis sechs Ringgliedern (für M = C) ergibt,

$R^4$ = H, F, -CN, $-CCl_3$, -C≡CH, ($C_1$-$C_4$)Alkyl, - 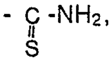

$R^5$ = Pyridyl, Furyl, Thienyl, die alle substituiert sein können, Phthalimidyl, De($C_1$-$C_4$)-alkylmaleinimidyl, Thiophthalimidyl, Dihydrophthalimidyl, Tetrahydrophthalimidyl, substituiertes Phenyl
oder $R^4$ und $R^5$ - zusammen mit dem sie verbrückenden Kohlenstoffatom - einen gegebenenfalls substituierten Indanyl-, Cyclopentenoyl- oder Cyclopentenyl-Rest
bedeuten, sowie deren für landwirtschaftliche Zwecke einsetzbaren Salze und Quaternisierungsprodukte.

2. Verbindungen der Formel I von Anspruch 1, worin
R' einen Rest der allgemeinen Formeln (A), (B), (C), (D), (E) oder (F),

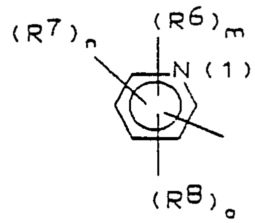

(A)

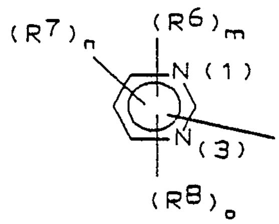

(B)

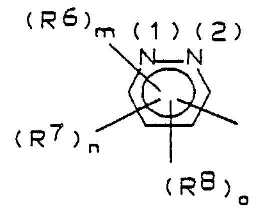

(C)

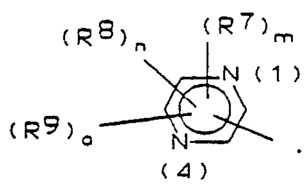

(D)

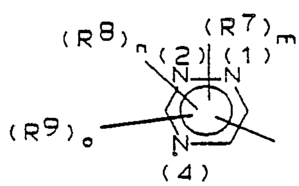

(E)

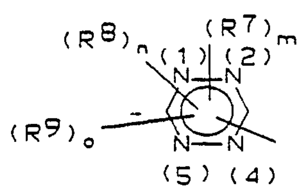

(F)

worin O ≦ m+n+o ≦ 3 und m, n, o die Werte 0 bis 2 besitzen können. $R^6$, $R^7$, $R^8$ und $R^9$ stehen unabhängig voneinander für ($C_1$-$C_5$)Alkyl, Halogen, ($C_2$-$C_6$)Alkenyl, ($C_2$-$C_6$)Alkinyl, ($C_3$-$C_7$)Cycloalkyl, ($C_1$-$C_3$)Alkoxy, ($C_2$-$C_4$)Alkenyloxy, ($C_2$-$C_4$)Alkinyloxy, ($C_1$-$C_4$)Alkylthio, ($C_3$-$C_6$)Cycloalkyloxy, ($C_1$-$C_4$)-Halogenalkyl, ($C_1$-$C_3$)Halogenalkoxy, ($C_1$-$C_3$) Halogenalkylthio, ($C_2$-$C_5$)Halogenalkenyl, ($C_2$-$C_5$) Halogenalkenyloxy, ($C_2$-$C_5$)Halogenalkenylthio oder zwei der Reste $R^6$, $R^7$, $R^8$, $R^9$ bilden - wenn sie orthoständig angeordnet sind - einen Methylendioxy-, Ethylendioxy oder ($C_3$-$C_5$)Alkylenrest und
$R^5$ einen Rest der Formel (G)

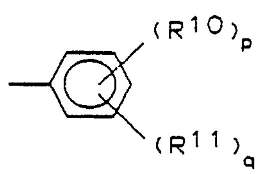

(G),

mit p, q = 1, $R^{10}$ = H oder 4-Fluor und $R^{11}$ in 3-Stellung des Phenylrests einen Rest der Formel (H)

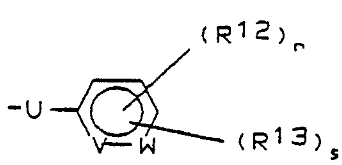

(H),

worin $R^{12}$ und $R^{13}$ = unabhängig voneinander H, Halogen, $(C_1\text{-}C_4)$Alkyl, $(C_1\text{-}C_4)$Alkoxy oder $(C_1\text{-}C_4)$-Halogenalkyl; U = 0 und V, W = CH bedeuten, oder
$R^5$ einen Rest der Formel (K)

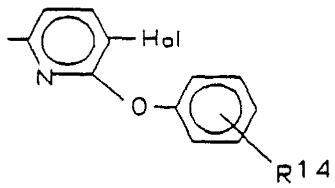

(K),

worin $R^{14}$ = Halogen außer J, $(C_1\text{-}C_4)$Alkyl, $(C_1\text{-}C_4)$Alkoxy oder $(C_1\text{-}C_4)$-Halogenalkyl und Hal = Fluor oder H bedeutet,
oder $R^5$ einen Rest der Fromel (L)

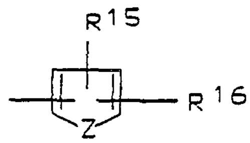

(L)

worin Z = O, S,
$R^{15}$ = H, Halogen, $(C_1\text{-}C_4)$Alkyl, $(C_1\text{-}C_4)$Alkoxy, $(C_1\text{-}C_4)$Halogenalkyl, CN oder $NO_2$ und
$R^{16}$ = Phenoxy
bedeutet, bedeuten.

3. Verbindungen der Formel I gemäß Anspruch 1 oder 2, worin $R^1$ einen Rest der Formel A bedeutet.

4. Verfahren zur Herstellung von Verbindungen der Formel I von Ansprüchen 1, 2, oder 3, dadurch gekennzeichnet, daß man
a) ein Mercaptan der allgemeinen Formel (II) oder dessen Salz der allgemeinen Formel III,

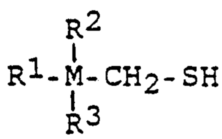

(II)

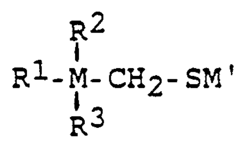

(III)

worin M′ einem Alkalimetall- oder Erdalkalimetall-Äquivalent, insbesondere Li, Na, K, Mg, Ca entspricht mit einem Alkylierungsmittel der allgemeinen Formel (IV),

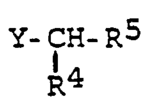

(IV)

worin Y eine nucleofuge Abgangsgruppe wie beispielsweise Halogen oder Sulfonat bedeutet, gegebenenfalls in Gegenwart einer Base,
oder
b) ein Mercaptan der allgemeinen Formel (V) oder dessen Salz der allgemeinen Formel (VI)

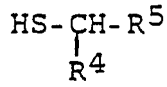

(V)

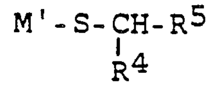

(VI)

mit einem Alkylierungsmittel der allgemeinen Formel (VII),

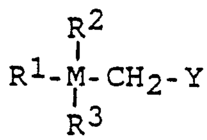

(VII)

gegebenenfalls in Gegenwart einer Base,
oder
c) ein Sulfid der allgemeinen Formel (VIII) oder (IX)

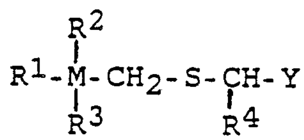

(VIII)

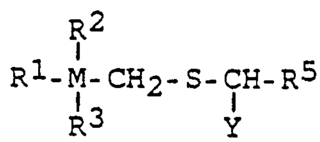

(IX)

mit einer Metallverbindung der allgemeinen Formel (X) oder (XI)

$M'-R^5$

(X)

$M'-R^4$

(XI)

oder - für M = Si -
d) ein Silan der allgemeinen Formel (XII)

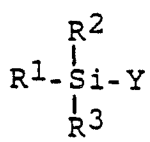

(XII)

mit einer metallorganischen Verbindung der allgemeinen Formel (XIII)

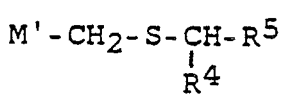

(XIII)

oder - für M = Si -
e) eine metallorganische Verbindung der allgemeinen Formeln (XIV), (XV) oder (XVI)

$R^1-M'$ (XIV) $R^2M'$ (XV) $R^3M'$ (XVI)

mit einem Silan der allgemeinen Formeln (XVII), (XVIII) oder (XIX)

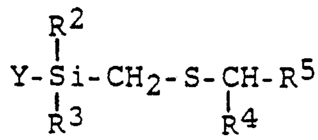 (XVII)

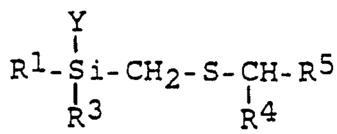 (XVIII)

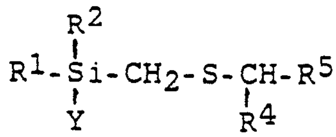 (XIX)

umsetzt.

5. Schädlingsbekämpfungsmittel, dadurch gekennzeichnet, daß sie eine Verbindung der Formel I von Ansprüchen 1, 2 oder 3 enthalten.

6. Insektizide Mittel, dadurch gekennzeichnet, daß sie eine Verbindung der Formel I von Ansprüchen 1, 2 oder 3 enthalten.

7. Akarizide Mittel, dadurch gekennzeichnet, daß sie eine Verbindung der Formel I von Ansprüchen 1, 2 oder 3 enthalten.

8. Nematozide Mittel, dadurch gekennzeichnet, daß sie eine Verbindung der Formel I von Ansprüchen 1, 2 oder 3 enthalten.

9. Verwendung von Verbindungen der Formel I von Ansprüchen 1,2 oder 3 als Schädlingsbekämpfungsmittel.

10. Verfahren zur Bekämpfung von Schadinsekten, Akariden oder Nematoden, dadurch gekennzeichnet, daß man auf diese, die von Ihnen befallenen Flächen, Pflanzen oder Substrate eine wirksame Menge einer Verbindung der Formel I von Ansprüchen 1, 2 oder 3 appliziert.

Patentansprüche für folgenden Vertragsstaat:ES

1. Verfahren zur Herstellung von Verbindungen der Formel I, ihre optischen Isomeren und deren Gemische,

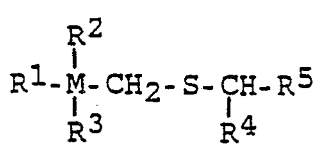 (I),

worin
M = C oder Si,
$R^1$ = unsubstituiertes oder substituiertes Pyridyl, unsubstituiertes oder substituiertes Pyrimidyl, sowie - für M = C - unsubstituiertes oder substituiertes Pyridazinyl, unsubstituiertes oder substituiertes Pyrazinyl, unsubstituiertes oder substituiertes 1,2,4-Triazinyl, unsubstituiertes oder substituiertes 1,2,4,5-Tetrazinyl,
$R^2$, $R^3$ = unabhängig voneinander $(C_1-C_3)$Alkyl, $(C_2-C_8)$ Alkenyl, $(C_1-C_2)$Haloalkyl, Phenyl oder $R^2$ und $R^3$

eine Alkylenkette, die - zusammen mit dem quartären Zentralatom (M) einen unsubstituierten oder fluorsubstituierten Ring mit vier bis sechs Ringgliedern (für M = Si) oder mit drei bis sechs Ringgliedern (für M = C) ergibt,

$R^4$ = H, F, -CN, $-CCl_3$, -C≡CH, $(C_1-C_4)$Alkyl, $-\underset{\underset{S}{\parallel}}{C}-NH_2$,

$R^5$ = Pyridyl, Furyl, Thienyl, die alle substituiert sein können, Phthalimidyl, De$(C_1-C_4)$-alkylmaleinimidyl, Thiophthalimidyl, Dihydrophthalimidyl, Tetrahydrophthalimidyl, substituiertes Phenyl

oder $R^4$ und $R^5$ - zusammen mit dem sie verbrückenden Kohlenstoffatom - einen gegebenenfalls substituierten Indanyl-, Cyclopentenoyl- oder Cyclopentenyl-Rest

bedeuten, sowie deren für landwirtschaftliche Zwecke einsetzbaren Salze und Quaternisierungsprodukte, dadurch gekennzeichnet, daß man

a) ein Mercaptan der allgemeinen Formel (II) oder dessen Salz der allgemeinen Formel III,

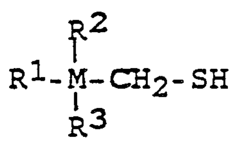

(II)

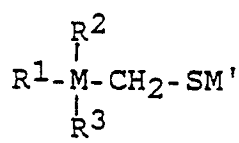

(III)

worin M′ einem Alkalimetall- oder Erdalkalimetall-Äquivalent, insbesondere Li, Na, K, Mg, Ca entspricht mit einem Alkylierungsmittel der allgemeinen Formel (IV),

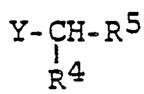

(IV)

worin Y eine nucleofuge Abgangsgruppe wie beispielsweise Halogen oder Sulfonat bedeutet, gegebenenfalls in Gegenwart einer Base,

oder

b) ein Mercaptan der allgemeinen Formel (IV) oder dessen Salz der allgemeinen Formel (IV)

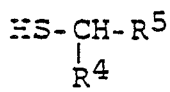

(V)

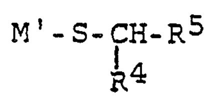

(VI)

mit einem Alkylierungsmittel der allgemeinen Formel (VII),

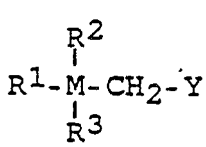

(VII)

gegebenenfalls in Gegenwart einer Base,

oder

c) ein Sulfid der allgemeinen Formel (VIII) oder (IX)

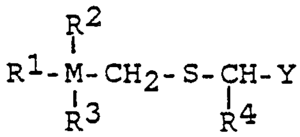

(VIII)

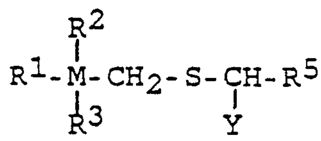

(IX)

mit einer Metallverbindung der allgemeinen Formel (X) oder (XI)

$M'-R^5$ (X)

$M'-R^4$ (XI)

oder - für M = Si -

d) ein Silan der allgemeinen Formel (XII)

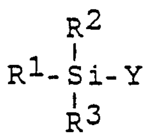

(XII)

mit einer metallorganischen Verbindung der allgemeinen Formel (XIII)

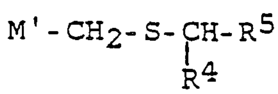

(XIII)

oder - für M = Si -

d) eine metallorganische Verbindung der allgemeinen Formeln (XIV), (XV) oder (XVI)

$R^1-M'$ (XIV)

$R^2M'$ (XV)

$R^3M'$ (XVI)

mit einem Silan der allgemeinen Formeln (XVII), (XVIII) oder (XIX)

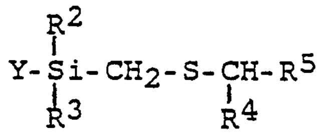

(XVII)

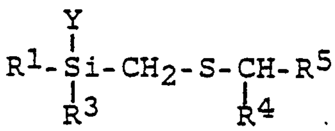

(XVIII)

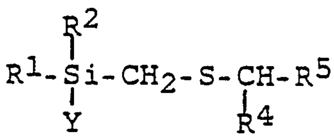

(XIX)

umsetzt.

2. Verfahren gemäß Anspruch 1, dadurch gekennzeichnet, daß in Formel 1 $R^1$ einen Rest der allgemeinen Formeln (A), (B), (C), (D), (E) oder (F),

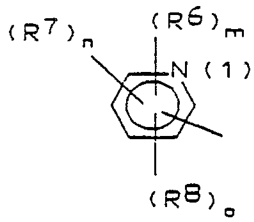

(A)

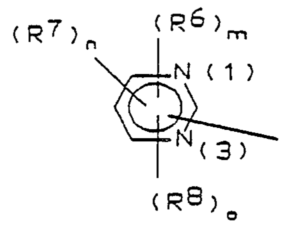

(B)

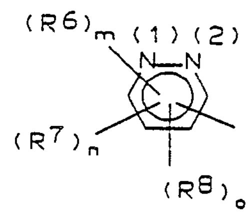

(C)

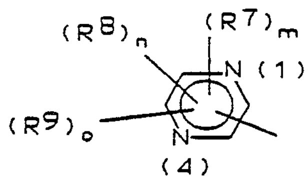

(D)

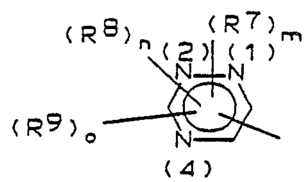

(E)

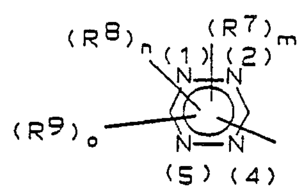

(F)

worin $0 \leq m+n+o \leq 3$ und m, n, o die Werte 0 bis 2 besitzen können. $R^6$, $R^7$, $R^8$ und $R^9$ stehen unabhängig voneinander für $(C_1-C_5)$Alkyl, Halogen, $(C_2-C_6)$Alkenyl, $(C_2-C_6)$Alkinyl, $(C_3-C_7)$Cycloalkyl, $(C_1-C_3)$Alkoxy, $(C_2-C_4)$Alkenyloxy, $(C_2-C_4)$Alkinyloxy, $(C_1-C_4)$Alkylthio, $(C_3-C_6)$Cycloalkyloxy, $(C_1-C_4)$-Halogenalkyl, $(C_1-C_3)$Halogenalkoxy, $(C_1-C_3)$ Halogenalkylthio, $(C_2-C_5)$Halogenalkenyl, $(C_2-C_5)$ Halogenalkenyloxy, $(C_2-C_5)$Halogenalkenylthio oder zwei der Reste $R^6$, $R^7$, $R^8$, $R^9$ bilden - wenn sie orthoständig angeordnet sind - einen Methylendioxy-, Ethylendioxy oder $(C_3-C_5)$Alkylenrest und

$R^5$ einen Rest der Formel (G)

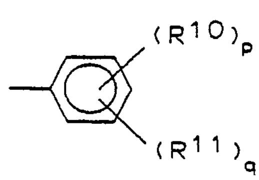

(G),

mit p, q = 1, $R^{10}$ = H oder 4-Fluor und $R^{11}$ in 3-Stellung des Phenylrests einen Rest der Formel (H)

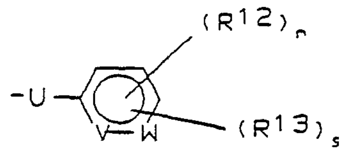

(H),

worin $R^{12}$ und $R^{13}$ = unabhängig voneinander H, Halogen, $(C_1-C_4)$Alkyl, $(C_1-C_4)$Alkoxy oder $(C_1-C_4)$-Halogenalkyl; U = 0 und V, W = CH bedeuten, oder
$R^5$ einen Rest der Formel (K)

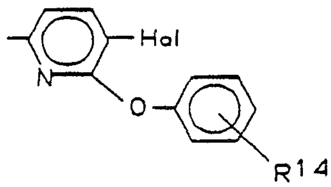

(K),

worin $R^{14}$ = Halogen außer J, $(C_1-C_4)$Alkyl, $(C_1-C_4)$Alkoxy oder $(C_1-C_4)$-Halogenalkyl und Hal = Fluor oder H bedeutet,
oder $R^5$ einen Rest der Fromel (L)

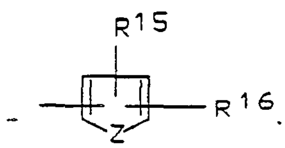

(L)

worin Z = O, S,
$R^{15}$ = H, Halogen, $(C_1-C_4)$Alkyl, $(C_1-C_4)$Alkoxy, $(C_1-C_4)$Halogenalkyl, CN oder $NO_2$ und
$R^{16}$ = Phenoxy
bedeutet, bedeuten.

3. Verfahren gemäß Anspruch 1 oder 2, dadurch gekennzeichnet, daß in Formel I $R^1$ einen Rest der Formel A bedeutet.

4. Schädlingsbekämpfungsmittel, dadurch gekennzeichnet, daß sie eine Verbindung der Formel I von Ansprüchen 1, 2 oder 3 enthalten.

5. Insektizide Mittel, dadurch gekennzeichnet, daß sie eine Verbindung der Formel I von Ansprüchen 1, 2 oder 3 enthalten.

6. Akarizide Mittel, dadurch gekennzeichnet, daß sie eine Verbindung der Formel I von Ansprüchen 1, 2 oder 3 enthalten.

7. Nematozide Mittel, dadurch gekennzeichnet, daß sie eine Verbindung der Formel I von Ansprüchen 1, 2 oder 3 enthalten.

8. Verwendung von Verbindungen der Formel I von Ansprüchen 1, 2 oder 3 als Schädlingsbekämpfungsmittel.

9. Verfahren zur Bekämpfung von Schadinsekten, Akariden oder Nematoden, dadurch gekennzeichnet, daß man auf diese, die von Ihnen befallenen Flächen, Pflanzen oder Substrate eine wirksame Menge einer Verbindung der Formel I von Ansprüchen 1, 2 oder 3 appliziert.

Europäisches Patentamt

# EUROPÄISCHER RECHERCHENBERICHT

Nummer der Anmeldung

EP 89 10 4995

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 249 015 (HOECHST AG)<br>* Seiten 31-58; Ansprüche 5-10 *<br>--- | 1-3,5-10 | C 07 D 213/32<br>C 07 D 213/64<br>C 07 D 213/70<br>C 07 D 213/65<br>C 07 D 239/34<br>C 07 D 239/38<br>C 07 D 237/14<br>C 07 D 237/18<br>C 07 D 253/06<br>C 07 D 257/08<br>C 07 D 409/12<br>C 07 D 491/056<br>C 07 F 7/08<br>A 01 N 55/00<br>A 01 N 43/40<br>A 01 N 43/54<br>A 01 N 43/58 |
| D,Y | EP-A-0 224 024 (HOECHST AG)<br>* Seite 29, Verbindung 57; Seite 39, Verbindung 117; Seite 41, Verbindung 129; Seiten 43,44, Beispiele; Ansprüche 4-9 *<br>--- | 1-3,5-10 | |
| A,P | EP-A-0 288 810 (HOECHST AG)<br>* Ansprüche; Seiten 119,120, Beispiele; Tabellen 1,2 *; & DE - A - 3712752 (Cat. D,A,P)<br>----- | 1-3,5-7,9,10 | |
| | | | **RECHERCHIERTE SACHGEBIETE (Int. Cl.5)** |
| | | | A 01 N 43/00<br>A 01 N 55/00<br>C 07 D 213/00<br>C 07 D 237/00<br>C 07 D 239/00<br>C 07 D 253/00<br>C 07 D 257/00<br>C 07 D 409/00<br>C 07 D 491/00<br>C 07 F 7/00 |

Der vorliegende Recherchenbericht wurde für alle Patentansprüche erstellt

| Recherchenort | Abschlußdatum der Recherche | Prüfer |
|---|---|---|
| BERLIN | 21-06-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)